(19) Europäisches Patentamt / European Patent Office / Office européen des brevets

(11) **EP 1 453 857 B1**

(12) **EUROPEAN PATENT SPECIFICATION**

(45) Date of publication and mention
of the grant of the patent:
**13.08.2014 Bulletin 2014/33**

(51) Int Cl.:
***C07K 14/505*** *(2006.01)*

(21) Application number: **02803796.8**

(22) Date of filing: **26.11.2002**

(86) International application number:
**PCT/EP2002/013299**

(87) International publication number:
**WO 2003/045996 (05.06.2003 Gazette 2003/23)**

(54) **CHROMATOGRAPHIC PURIFICATION OF RECOMBINANT HUMAN ERYTHROPOIETIN**

CHROMATOGRAFISCHE AUFREINIGUNG VON RECOMBINANTER HUMANER ERYTHROPOIETIN

PURIFICATION CHROMATOGRAPHIQUE D'ERYTHROPOIETINE HUMAINE RECOMBINEE

(84) Designated Contracting States:
**AT BE BG CH CY CZ DE DK EE ES FI FR GB GR IE IT LI LU MC NL PT SE SK TR**
Designated Extension States:
**LT LV RO SI**

(30) Priority: **28.11.2001 US 333839 P**

(43) Date of publication of application:
**08.09.2004 Bulletin 2004/37**

(73) Proprietor: **Sandoz AG**
**4056 Basel (CH)**

(72) Inventors:
• **ALLIGER, Peter**
**A-6330 Kufstein (AT)**
• **PALMA, Norbert**
**A-6252 Breitenbach a. Inn (AT)**

(56) References cited:
**US-A- 5 856 298**

• **WATSON ET AL: "Capillary electrophoretic separation of human recombinant erythropoietin (r-HuEPO) glycoforms" ANALYTICAL BIOCHEMISTRY, vol. 210, - 1993 pages 389-393, XP002231297**

**Description**

Field of the Invention

The present invention relates to a procedure for the production of recombinant human erythropoietin (Epo) with a defined composition of glycoforms in a highly pure form, i.e. with a low amount of host cell proteins. This is achieved by a specific sequence of purification steps in combination with an analytical tool to quantify the separated isoforms.

Background to the invention

Erythropoietin is the principal hormone regulating the proliferation and differentiation of erythroid progenitor cells and the maintenance of physiological levels of circulating red blood cells. In the fetus Epo is primarily produced in the liver and about 90% of its production switches to the kidney after birth. When Epo levels fall due to chronic or acute renal failure, Epo must be externally administered to prevent a rising anemia. A therapeutically active human erythropoietin has been available since the discovery of the Epo gene and its expression in rodent cells.

The human Epo gene encodes a 27 amino acid signal peptide and a 166 amino acid protein with a calculated molecular weight of 18396 Dalton. The mature protein usually has a one amino acid N-terminal deletion, and is 165 amino acids in length. The signal sequence directs the peptide to the cellular compartments involved in the proper glycosylation, leading to a mature protein with three N- and one O-glycosylation site. The sugar moiety, which makes about 40% of the total molecular weight, is essential for the full biological activity of Epo. Several studies have shown that the number of terminal sialic acid residues have an positive effect on the *in vivo* half-life, although the *in vitro* activity, i.e. the binding to the receptor, is highest in the non or partly glycosylated form (Takeuchi and Kobata, 1991, Glycobiology 1 (4): 337-346). The degree on sialylation is directly proportional to the half-life, where the isoforms with less sialic acids are much faster cleared from the organism and therefore show less activity.

The objective in generating a highly active recombinant erythropoietin is to create a product with a high degree of terminal sialic acid residues by a well optimized fermentation strategy and by a selective purification protocol.

There are numerous publications on the purification of Epo from different sources. Before the cloning of erythropoietin and use of recombinant DNA technology, most of the described purifications used human urine as a natural source. All of the following protocols for Epo purification end up with a pure product with no emphasis on the accumulation of defined isoforms. T. Miyake et al., 1977, J. Biol. Chem 252(15): 5558-5564 describe the purification of urinary Epo by a seven step procedure, including ion-exchange chromatography, ethanol precipitation, gel filtration and adsorption chromatography. A different procedure is described by N. Inoue et al., 1994, Biol. Pharm. Bull., 17(2): 180-184 using anion-exchange chromatography, gel filtration, lectin chromatography and reversed phase chromatography to purify Epo from human urine. Urine is also the source for the five step purification of G. Krystal et al., 1986, Blood, 67(1): 71-79 with Affi-Blue, chromatofocusing, lectin-chromatography, reversed phase chromatography and preparative SDS-PAGE. A different combination of the above stated purification steps is published by H. Yianagi et al., 1987, J. Chromatogr.417: 178-182. Their downstream-processing (DSP) protocol started with urine purified by ethanol precipitation, two lectin-chromatography steps, hydroxylapatite and reversed phase chromatography.

V. Broudy et al., 1988, Arch. Biochem. Biophys. 265(2): 329-336 used a transfected BHK cell line to purify erythropoietin by Affi-Gel blue chromatography, anion-exchange chromatography and reversed-phase chromatography. The purified Epo is not enriched for specific isoforms.

A.B. Ghanem, 1994, Prep. Biochem. 24(2): 127-142 and A. Gokana et al., 1997, J. Chromatogr. 791: 109-118 used a DEAE-Sephacel to separate isoforms of erythropoietin generated in a B-lymphoblastoid cell line after an affinity chromatography step. They analyzed the purified product by isoelectric focusing, but did not pool fractions with a defined isoform.

J. Burg et al., WO99/28346 purified Epo to a high degree on N-acetyl-lactosamine units and/or tetra-antenna branches in the carbohydrate structure. The DSP sequence starts with a cell culture supernatant, capture by affinity chromatography, purification by hydrophobic interaction chromatography, hydroxylapatite and reversed phase chromatography. Drawbacks of this procedure are a possible leakage of the Cibacron Blue 3G dye-ligand of the blue-sepharose capture matrix, a rather weak separation of host cell proteins by the HIC step and a silica based RPC resin, which is not stable to sanitation by NaOH.

Summary of the Invention

The present invention provides a method for recovering and purifying recombinant human erythropoietin (rhEpo) from a cell culture medium comprising host cells, which method comprises the steps of:

(a) removing host cells, cellular constituents and debris from the cell culture medium by performing a procedure selected from the group consisting of (i) centrifugation followed by a depth filtration step, (ii) a depth filtration step, and (iii) centrifugation, to obtain a clarified culture medium supernatant;

(b) adjusting the conductivity of the supernatant to 5 mS/cm or less, and a pH of between about 7.0 and 8.0;

(c) applying the supernatant from step (b) to a column comprising an anion exchange chromatographic medium, washing the column, eluting the rhEpo from the column, and collecting the peak fraction(s) that contain rhEpo;

(d) subjecting the combined peak fractions from step (c) to a reverse phase chromatography step using a resin that can be run under medium pressure (< 10 bar) and is resistant to high concentrations of NaOH, the rhEpo being eluted using a linear gradient of an organic solvent;

(e) applying one or more fractions eluted in step (d) which contain rhEpo to a column comprising anion exchange chromatographic media, washing the column, and eluting the rhEpo using a linear salt gradient;

(f) selecting one or more fractions eluted in step (e) which contain rhEpo based on degree of sialylation of the rhEpo; and

(g) subjecting one or more fractions eluted in step (f) which contain rhEpo by one or more size exclusion chromatographic steps using a gel filtration medium to remove potential dimers and higher aggregates; and collecting the eluate containing rhEpo.

## Detailed Description of the invention

A new purification protocol has been developed that makes use of modern, polymeric resins, which are rigid and withstand harsh cleaning in place procedures including 1 M NaOH or acetic acid. Furthermore, these resins have high binding capacities and allow high flow rates for an efficient process at production scale. Resins with potentially leakable ligands, such as lectin or dyes, which could pose problems to patients are not used.

The implemented purification steps have different selectivities leading to a very pure product with low amounts of proteins or DNA from the host cell. Further, in the purification process according to the present invention as outlined herein, several, preferably at last three, of the chromatographic steps have the potential to separate the individual isoforms. In particular, the fractions of the second anion-exchange chromatography step (i.e. step (e), see below), may be analyzed by CZE [capillary zone electrophoresis] before pooling and further processing by final gel filtration chromatography. In the alternative, or additionally, the fractions of the reverse phase chromatography step (i.e. step (d), see below) may be analyzed by CZE and treated accordingly. Another possibility is to perform (alternatively or additionally) a CZE with the fractions obtained by performing the first anion-exchange chromatography, i.e. step (c), see below. Using CZE, glycosylated proteins or polypeptides, which are contained as a mixture or composition of isoforms in the fraction(s) to be analyzed, are separated into the specific isoforms. By comparison with known isoform standards, in particular standards with respect to the glycosylation pattern, e.g. the sialylation pattern, it is possible to assign a specific structure to each isoform separated by CZE. In the context of the present invention, this results in a fraction pool with a defined composition of highly glycosylated isoforms independent of the quality of the source. More generally speaking, in the course of the present invention it has been established that CZE is a valuable tool in the production of proteinaceous compositions which comprise glycosylated isoforms of a protein or polypeptide, independently of the nature of the protein or polypeptide itself. Accordingly, the present invention provides the use of CZE as an analytical tool in the production of a defined composition of isoforms of a glycosylated protein or polypeptide. In particular, such use can be favourably applied in the production of a defined isoform composition of recombinant human erythropoietin. The present invention therefore comprises a method for production of a defined isoform composition of a glycosylated protein or polypeptide, in particular of recombinant human erythropoietin, said method comprising analyzing samples (which may be fractions of a chromatographic purification step) comprising one or more isoforms of a glycosylated protein or polypeptide using CZE, and, where desired, pooling such samples which contain the desired isoform(s) of such glycosylated protein or polypeptide, in particular wherein said isoforms of the glycosylated protein or polypeptide are isoforms of recombinant human erythropoietin.

Furthermore, this process is designed to release only very low amounts of cellular proteases during primary separation by reducing shear stress and cell lysis, and using conditions where residual proteases are not active and harmful to the product. This is achieved firstly, by a cell separation with hermetically designed disc stack centrifuges to keep the cells intact and second, by a capture step using anion-exchange chromatography, which works at neutral pH. A pH below 6 would result in cleavage by an endogenous low pH active protease.

Finally, several steps in the downstream processing (DSP) sequence are robust in virus removal and/or inactivation. This is an important requirement for mammalian cell derived therapeutics, as viral contamination can not be ruled out. The implemented nano-filtration especially helps to remove even very small and non-enveloped viruses, such as parvoviruses. The solvent exposure during and/or after reversed phase chromatography is another robust step leading to inactivation of enveloped viruses.

Accordingly, the present invention provides a method for recovering and purifying recombinant human erythropoietin (rhEpo) from a cell culture medium comprising host cells, which method comprises the steps of:

(a) removing host cells, cellular constituents and debris from the cell culture medium by performing a procedure selected from the group consisting of (i) centrifugation followed by a depth filtration step, (ii) a depth filtration step, and (iii) centrifugation, to obtain a clarified culture medium supernatant;

(b) adjusting the conductivity of the supernatant to 5 mS/cm or less, and a pH of between about 7.0 and 8.0;

(c) applying the supernatant from step (b) to a column comprising an anion exchange chromatographic medium, washing the column, eluting the rhEpo from the column, and collecting the peak fraction(s) that contain rhEpo;

(d) subjecting the combined peak fractions from step (c) to a reverse phase chromatography step using a resin that can be run under medium pressure (< 10 bar) and is resistant to high concentrations of NaOH, the rhEpo being eluted using a linear gradient of an organic solvent;

(e) applying one or more fractions eluted in step (d) which contain rhEpo to a column comprising anion exchange chromatographic media, washing the column, and eluting the rhEpo using a linear salt gradient;

(f) selecting one or more fractions eluted in step (e) which contain rhEpo based on degree of sialylation of the rhEpo; and

(g) subjecting one or more fractions eluted in step (f) which contain rhEpo by one or more size exclusion chromatographic steps using a gel filtration medium to remove potential dimers and higher aggregates; and collecting the eluate containing rhEpo.

Preferably, the centrifugation in step (a) is performed using a disc stack separator.

In a preferred embodiment, the conductivity in step (b) is adjusted to between 2 and 5 mS/cm, preferably to about 3 mS/cm.

Advantageously, the anion exchange medium used in step (c) is the ceramic-based ion exchange medium Q-HyperD F™, obtainable from BioSepra. The resin used in step (d) advantageously is a resin which is resistant to a concentration of NaOH of about 0.5 M; preferably it is a polystyrene resin, and advantageously it is Source 30RPC™ (obtainable from Amersham Biosciences), whilst the anion exchange medium used in step (e) is preferably a sepharose anion exchange chromatographic media, and advantageously it is Q Sepharose High Performance™ (obtainable from Amersham Biosciences). The gel filtration medium used in step (g) is preferably Superdex 75 prep grade™ (obtainable from Amersham Biosciences).

In a preferred embodiment, prior to step (d), the peak fractions from step (c), which preferably may be combined, are subjected to an ammonium sulfate precipitation step to precipitate contaminating host cell proteins, the ammonium sulfate concentration of the supernatant then being adjusted to a concentration compatible with step (d). Before loading the reversed phase column of step (d), such concentration preferably is less than 0.24 M ammonium sulfate.

Typically, the pH is adjusted in step (b) to about pH 7.5. Preferably, the washing in step (c) is performed with an appropriate aqueous buffer, e.g. a Tris buffer, comprising a salt, like NaCl. In a preferred embodiment, a 20 mM Tris buffer with a pH of 7.5, comprising 50 mM NaCl, is used. The elution is likewise performed with an appropriate aqueous buffer, e.g. a Tris buffer, comprising a salt, like NaCl. The elution step in step (c) is generally performed using a buffer having a salt concentration between 100 mM and 1 M, in particular of greater than 125 mM, preferably 150 mM. In a preferred embodiment, a 20 mM Tris buffer with a pH of 7.5, comprising 150 mM NaCl, is used in this elution step. The organic solvent in step (d) preferably is selected from the group consisting of acetonitrile, ethanol and hexylene glycol (i.e. 2-methyl-2,4-pentanediol), acetonitrile being most preferred. Preferably, before applying fractions comprising erythropoetin to the reverse phase chromatography, the fractions are diluted with an aqueous buffer, like 20 mM Tris/HCl, pH 7.0, comprising the organic solvent, preferably acetonitrile. After applying the diluted fractions onto the reverse phase column, preferably the column is washed with an aqueous buffer, like 20 mM Tris/HCl, pH 7.0, comprising the organic solvent, preferably acetonitrile. Preferably, in step (d) the Epo polypeptide is eluted employing a linear gradient of the organic solvent of from about 10% to about 100%. In a particularly preferred embodiment thereof, in step (d) a linear gradient of acetonitrile typically from about 25% to 50% is employed for elution. The linear salt gradient in step (e) is typically from 0 to 300 mM. Preferably, the salt in steps (c) and (e) is NaCl.

In one embodiment of the present invention, the fractions eluted in step (d), which comprise the organic solvent, preferably acetonitrile, are diluted with an appropriate aqueous buffer, which is compatible with the following anionic exchange chromatographic step. In order to inactivate viral contaminants, prior to step (e) the diluted fractions are left for an appropriate amount of time which is sufficient to achieve the desired inactivation of such viral contaminants. For example, the fraction can be diluted with 0.5 of its volume with the aqueous buffer (in particular 20 mM Tris/HCl, pH 7,0) and incubated for at least about 20 min. up to about 40 min., or even longer, and then further diluted with 3.5 volumes of the fraction's original volume with the aqueous buffer.

In a preferred embodiment of the present invention, in step (a) any of the procedures (i), (ii) or (iii) is followed by a sterile filtration step. Typically, this is performed by using a 0.2 $\mu$m filtration unit.

In a further embodiment of the present invention, during the process of the present invention fractions derived form a chromatographic column may be subjected to an ultrafiltration step, in particular for concentration. Accordingly, in a preferred embodiment, in step (g) the fractions are subjected to an ultra-filtration step prior to the size exclusion chromatography step,. In another preferred embodiment, in step (d) the fractions are subjected to an ultrafiltration step prior to reverse phase chromatography. In a likewise preferred embodiment, if the process of the present invention comprises an ammonium sulfate precipitation step, as described above, the peak fractions from step (c), single or combined, are subjected to an ultrafiltration step prior to such ammonium sulfate precipitation step. Preferably, all of the three ultrafiltration steps mentioned herein are employed in the process of the present invention. For example, a membrane with a cutoff of between 5 kDa and 10 kDa, for example 10 kDA, preferably with a 5 kDa cutoff, may be used in such ultrafiltration process.

In a preferred embodiment, a dead-end nano-filtration step is included to remove viruses before or, preferably, after step (g). Examples of units include the Planova 15N (Asahi), PALL Ultipor VF Grade DV20 or Millipore Viresolve NFP cartridges or capsules.

As discussed above, it is desirable to be able to select for preferred isoforms during the purification steps and this can be achieved using the purification method of the invention. Specifically, this can be achieved because the different isoforms are resolved during both the reverse phase chromatography step and the second anion exchange chromatography step. The content of different fractions can be determined by capillary zone electrophoresis as an in-process control, and selected fractions combined or discarded as appropriate. In preferred embodiments of the present invention, one or more of the selection procedure in step (f) or of the selection procedure between step (d) and step (e), as described above, is performed using capillary zone electrophoresis, as outlined above.

In the context of the present invention, the preferred or particular embodiments as described herein are capable of being combined with each other, thereby resulting in further preferred embodiments thereof.

Thus in a preferred embodiment, the present invention provides a method for recovering and purifying recombinant human erythropoietin (rhEpo) from a harvested cell culture , which method comprises the steps of:

(a) harvesting the cell culture and removing the host cells, cellular constituents and debris from the culture medium by performing a procedure selected from the group consisting of (i) centrifugation using a disc stack separator followed by a depth filtration step, (ii) a depth filtration step, and (iii) centrifugation, each followed by a 0,2 $\mu$m filtration step to obtain a clarified culture supernatant; and

(b) adjusting the conductivity of the supernatant to 5 mS/cm or less, and a pH of between about 7.0 and 8.0; and

(c) applying the supernatant from step (b) to an anion exchange column packed with Q-HyperD F , washing the column, eluting the rhEpo from the column, and collecting the peak fraction(s) that contain rhEpo; and

(d) subjecting the combined peak fractions from step (c) to a reverse phase chromatography step using a polystyrene resin that can be run under medium pressure (< 10 bar) and is resistant to NaOH CIP conditions such as Source 30RPC, the rhEpo being eluted using a linear gradient of acetonitrile; and

(e) selecting one or more fractions eluted in step (d) which contain rhEpo based on degree of sialylation of the rhEpo, applying said fractions to a high performance anion exchange column packed with Q-Sepharose HP , washing the column, and eluting the rhEpo using a linear salt gradient;

(f) selecting one or more fractions eluted in step (e) which contain rhEpo based on degree of sialylation of the rhEpo, subjecting said fractions to size exclusion chromatography using Superdex 75 prep grade to remove potential dimers and higher aggregates; and collecting the eluate containing rhEpo.

Preferably the determination as to which of the one or more fractions which are obtained in step (d) and in step (e), respectively, are to be selected in step (e) and/or step (f), respectively, for further chromatographic purification is performed by capillary zone electrophoresis. By way of guidance, it has been shown that the higher glycosylated forms of rhEpo are present in the first half of the elution peak in the RPC step, whereas in the Q Sepharose HP step the highly glycosylated and sialylated isoforms are present in the second half of the elution peak. As outlined above, in a preferred embodiment prior to step (d), the combined peak fractions from step (c) are subjected to an ammonium sulfate precipitation step to precipitate contaminating host cell proteins, the ammonium sulfate concentration of the supernatant then being adjusted to a concentration compatible with step (d). Preferably, said concentration is less than 0.24 M ammonium sulfate. Likewise preferred in this context, the preferred method of the present invention further comprises a dead-end nano-filtration step to remove viruses before or, preferably, after step (f).

In relation to the source material for the purification procedure, it is preferred that the host cells have been cultured in serum-free culture medium. It is also preferred that the host cells have been cultured using a discontinuous fed batch fermentation process. In particular, the host cells are capable of expressing recombinant human erythropoietin and secreting the erythropoietin into the culture medium. Host cells are generally mammalian cells. Preferred host cells are CHO cells.

The present invention also provides a composition comprising purified recombinant human erythropoietin produced by the methods of the invention. The present invention further provides purified recombinant human erythropoietin obtainable by the methods of the invention and a composition, in particular a pharmaceutical composition, comprising said purified erythropoietin.

Preferably, the composition has a defined content of glycosylation/sialylation isoforms of rhEpo. This may be achieved because the different forms are resolved during both the reverse phase chromatography step and the second anion exchange chromatography step. The content of different fractions can be determined by capillary zone electrophoresis as an in-process control, and selected fractions combined or discarded as appropriate. In particular, the first half of the elution peak is pooled from the RPC step since this is where the higher glycosylated forms are present. By contrast, the second half of the elution peak in the Q Sepharose HP step typically contains the highly glycosylated and sialylated isoforms.

Preferably in a composition comprising a recombinant polypeptide, particularly Epo, expressed in and purified using the method of the invention, the recombinant polypeptide is substantially pure, such at least 90%, 95%, 99% or 99.5% pure.

The biological activity of the purified protein can be determined *in vitro* and/or *in vivo*. A suitable *in vitro* test is described in Hammerling et al., 1996, J Pharm Biomed Anal 14(11):1455-69, which involves testing for proliferative stimulation of an erythroid cell line. A suitable *in vivo* test is described in Ghanem *et al.,* 1994, supra, which involves determining the incorporation of $^{59}$Fe into red blood cells of polycythemic mice.

In a preferred aspect the present invention can be conducted in conjunction with nucleic acid vectors and host cells in which (a) a first polynucleotide vector which comprises (i) a first nucleotide sequence which encodes a recombinant polypeptide of interest; and (ii) a second nucleotide sequence encoding a selectable marker, which second nucleotide sequence is amplified when the host cell is contacted with a selection agent, and (b) a second polynucleotide vector having essentially the same nucleotide sequence as the first polynucleotide vector except that the second nucleotide sequence is replaced with a third nucleotide sequence which encodes a different selectable marker; the first polynucleotide vector and second polynucleotide vector being integrated into the genome of the host cell.

Preferably the host cell is a mammalian cell, more preferably a Chinese hamster ovary (CHO) cell.

Preferably the second nucleotide sequence encodes a dihydrofolate reductase polypeptide and the selection agent is methotrexate. Preferably the recombinant polypeptide of interest is human erythropoietin. Such systems are described in the examples section of this document.

Cells are advantageously cultured by (a) providing a host cell which comprises a nucleotide sequence which encodes the recombinant polypeptide of interest and which directs expression of the recombinant polypeptide of interest in the host cell; (b) providing a serum-free culture medium which comprises (i) water, a plant-derived peptone, an osmolality regulator, a buffer, an energy source, amino acids, a lipid source or precursor, a source of iron, non-ferrous metal ions and one or more vitamins and cofactors; and (ii) does not contain any full-length polypeptides; and (c) culturing the host cell in the culture medium under conditions that allow for expression of the recombinant polypeptide of interest.

Preferably the recombinant polypeptide of interest is human erythropoietin. The host cell may be a Chinese hamster ovary (CHO) cell. Such techniques are described in the examples section of this document.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2nd ed. (1989) Cold Spring Harbor Laboratory Press, Cold Spring Harbor, N.Y. and Ausubel et al., Short Protocols in Molecular Biology (1999) 4th Ed, John Wiley & Sons, Inc. - and the full version entitled Current Protocols in Molecular Biology, which are incorporated herein by reference) and chemical methods.

The present invention is described further with reference to the following examples, which are illustrative only and non-limiting. In particular, the examples relate to preferred embodiments of the present invention.

## EXAMPLES

### *Materials*

#### Host cell line

Chinese hamster ovary (CHO) dihydrofolate-reductase deficient (ATCC CRL-9096). They have been deposited under the Budapest Treaty under deposit designation no. ATCC PTA-3672 at the American Type Culture Collection (ATCC), Rockville, Md. 20852, USA, on August 29, 2001.

#### Cell culture Media

| | |
|---|---|
| Cultivation medium: | DMEM supplemented with L-Glutamine, 4mM, 10% FCS, HT (Hypoxanthine, Thymidine), 1x |
| Selection medium: | DMEM supplemented with L-Glutamine, 4mM, dialyzed FCS, 10% and G418, 0.5 mg/ml |
| Amplification medium: | DMEM supplemented with L-Glutamine, 4mM, dialyzed FCS, 10%, G418, 0.5 mg/ml, and MTX (methotrexate), $4.8 \times 10^{-8}$M - $1.54 \times 10^{-6}$M |
| Freezing medium: | DMEM supplemented with L-Glutamine, 4mM, FCS, 10% and DMSO, 10%. |
| Serum-free adaptation medium | 1:1 DMEM/Ham's F12, supplemented with: |
| for recombinant cell-lines: | L-Glutamine, 6mM, Soya-peptone/UF, 0.25%, Hybridoma Supplement, 1 x, Pluronic-F68, 0.1%, G418, 0.5 mg/ml, MTX, $1.54 \times 10^{-6}$M |
| Serum-free production medium: | 1:1 DMEM/Ham's F12, supplemented with: |
| | Soya-peptone/UF, 0.25%, Hybridoma Supplement, 1x, Lutrol, 0.1 %, MTX, $1.54 \times 10^{-6}$M, Glucose 1g/l, $NaHCO_3$, 2.5g/l |
| Serum-free freezing medium | PBS, PVP-10, 20%, DMSO, 5% |
| for recombinant cell-lines: | Hybridoma Supplement, 100x Ethanolamine - $2.5 \times 10^{-3}$ M, Ferric-Citrate - $2.5 \times 10^{-2}$ M, L-Ascorbic Acid - $2.0 \times 10^{-3}$ M, Sodium Selenite - $5.0 \times 10^{-6}$ M |

Plasmid constructs

*pEpo/neo*

This plasmid encodes the coding region of Epo and the neomycin resistance gene as two different expression cassettes each under control of SV40 early promoter/terminator sequences. The construction details are provided in Example 1.

*pEpo/dhfr*

This plasmid encodes the coding region of Epo and dhfr as two different expression cassettes each under control of SV40 early promoter/terminator sequences. The construction details are provided in Example 2.

***Methods in cell culture***

Growth of CHO-dhfr⁻

Dihydrofolate reductase deficient CHO cells (Urlaub et al., 1980, PNAS 77(7):4216-4220) (referred to as CHO dhfr) are cultivated in DMEM cultivation medium with a splitting ratio 1:10 twice a week.

Transfection of CHO cells

$1\text{-}5 \times 10^4$ cells per $cm^2$ are seeded in $25cm^2$ T-flask bottles or 96-well plates the day before the lipofectin transfection is performed. The corresponding plasmids are mixed in the appropriate ratio, added to the lipofectin reagent (GIBCO/BRL) according to the manufacturer's protocol (0.5-1 $\mu$l/$cm^2$). Then the cells are overlaid with the transfection cocktail for four to sixteen hours in serum-free DMEM, before the DNA-containing medium is replaced with cultivation medium. After cultivation for 24 to 48 hours in the serum-containing medium the cells are switched to selection medium. Transfected cell pools are first cultivated in selection medium to confluence and then in amplification medium ($4.8 \times 10^{-8}$ M MTX) before screening the cell culture supernatants by ELISA for Epo production. Highest producers are determined, the MTX concentration increased two-fold and best producers used for further cultivation.

*Analytical methods*

ELISA detecting Epo in different matrices

**Antibodies**

polyclonal serum: rabbit anti Epo biotinylated (R&D Systems; Catalog # AB-286-NA) monoclonal antibody: mouse anti Epo (Genezyme; Cat. code AE7A5)

**ELISA buffers**

| | |
|---|---|
| Coating buffer: | 8.4 g/l $NaHCO_3$, 4.2 g/l $Na_2CO_3$, pH 9.6-9.8 |
| Washing buffer: | 0.2 g/l KCl, 1.15 g/l $Na_2HPO_4$ x $2H_2O$, 0.2 g/l $KH_2PO_4$, 8 g/l NaCl, 0.1 % Tween 20 |
| Dilution buffer: | 2% PVP (Sigma Cat.No. PVP-4OT) in washing buffer |
| Sample buffer: | 0.5% alpha mono-thio-glycerol in dilution buffer |
| Staining buffer: | 7.3 g/l Citric acid x $2H_2O$, 11.86 g/l $Na_2HPO_4$x$2H_2O$, pH 4.8-5.0 |
| Staining solution: | 100 $\mu$l OPD solution/10 ml staining buffer 5 $\mu$l $H_2O_2$/10 ml staining buffer |
| OPD stock-solution: | PP: L0017 |

**Method**

The ELISA method used detects Epo in ng/ml concentration ranges, in particular with a detection limit in the range of about 10 ng/ml, starting with 500 ng/ml and eight twofold dilutions. One monoclonal antibody against the first 26 amino acids of Epo functions as a coating layer, binding Epo. In the next step Epo is specifically bound by the catcher antibody. Detection is arranged through a biotinylated Epo recognizing rabbit antiserum. Visualization is performed by staining with OPD after streptavidin-peroxidase coupling to the plate.

Immunofluorescence

Epo expressing CHO-cells are inoculated with $5x10^4$ cells/200 $\mu$l on a cover slip in a 6-well plate and incubated for 24-72 hours. The adherent cells are washed twice with PBS and fixed for 5 minutes with -20°C methanol, then air dried and afterwards again soaked in PBS. Unspecific proteins are saturated by incubation with 20% FCS in PBS for 15 minutes and then anti-Epo is incubated for one hour. The reaction is visualized with anti-mouse IgG FITC conjugated. The fluorescence is detected by confocal microscopy at an excitation wavelength of 488 nm and an emission wavelength of higher then 515 nm.

Nucleus size determination

**Materials**

Coulter Counter® Model ZM (Coulter Electronics Inc.)
Coulter Channelyzer® Model 256
Incubation solution: Citric acid, 0.1 M, Triton X 100, 2%
Electrolyte Isoton II (Kat-No.844 8011; Coulter Euro Diagnostic GmbH)

The Coulter Counter quantifies particles in size and number, that are suspended in an electric conductive fluid. The fluid is absorbed by vacuum through a capillary which carries electrodes on both sides. The change of the resistance induces a voltage impulse that can be digitized by the Coulter Channelizer. Nucleus size correlates with DNA content of the cells, so that it is possible to distinguish between a diploid and a polyploid set of chromosomes.

**Method**

Approximately 1x10^6 CHO-cells are washed once in PBS, resuspended in 2 ml incubation solution and left there for 4-5 hours. The following steps are specific for the Coulter Counter.

SDS Polyacrylamide-Electrophoresis and Western blotting

**Materials**

| | |
|---|---|
| SDS Gels: | Novex Tris-Glycine 4-20% |
| Sample buffer: | Tris Glycine SDS 2x (Novex LC 2676) |
| Running buffer: | Tris Glycine SDS (Novex LC 2675) |
| Blotting buffer: | $Na_2B_4O_7$x10$H_2O$, 50 mM, SDS, 0.1 %, methanol, 20% |
| Blotting matrix: | PVDF Immobilon P 0.45$\mu$M Millipore; K8JM8238H |
| Washing buffer: | see ELISA washing buffer |
| Dilution buffer: | 1% milk powder in washing buffer |
| Detection buffer: | NaCl, 0.1 M |
| | Tris-HCl 0.1 M pH 9.5 |

**Method**

Epo containing samples are adjusted to 30 ng/20$\mu$l in 1x sample buffer with 1% $\alpha$-MTG and applied to the SDS gel. At the end of the run, the proteins are blotted to a PVDF immobilon membrane for two hours and then the Epo is specifically stained with a monoclonal antibody detecting the first 26 amino acids of Epo. Visualization is done with anti mouse IgG conjugated to alkaline phosphatase and NBT/BCIP staining.

Isoelectric focussing

**Materials**

| | |
|---|---|
| System: | Multiphor II, Amersham Biosciences |
| IPG Gels: | pH 2.5 -7 |
| Reswelling buffer: | 9 g urea, 0.5 g CHAPS, 0.04 g DTE, 0.5 ml resolytes (pH 3.5-10) 10 $\mu$l bromphenol-blue (0.1 %), adjust to 25 ml with $H_2O$ |
| Sample buffer: | IPG sample buffer pH 3-10, 25$\mu$l in 625 $\mu$l $H_2O$ |
| Blotting buffer: | 2.93 g Glycine, 5.81 g Tris, 20 ml methanol, 0.375 g SDS adjust to 1000 ml with $H_2O$ |
| Blotting matrix: | PVDF Immobilon P 0.45 $\mu$M Millipore; K8JM8238H |
| Washing buffer: | see ELISA washing buffer |
| Dilution buffer: | 1% milk powder in washing buffer |
| Detection buffer: | NaCl, 0.1 M, Tris-HCl 0.1 M pH 9.5 |

**Method**

Epo containing samples are adjusted to 500-1000 ng/50 $\mu$l, desalted, diluted 1:1 in sample buffer and applied to the reswollen IPG gel. Running conditions are first one minute 300V, then linear increase to 3500V and at the end 1 hour at 3500V. During the whole focussing process a limit of 10 mA and 10W is set. Afterwards the proteins are blotted to a PVDF Immobilon membrane by diffusion overnight or by electroblot and then Epo is stained specifically with a monoclonal antibody detecting the first 26 amino acids of Epo. Visualization is done with anti mouse IgG AP conjugated and NBT/BCIP staining.

Determination of the DNA content

The DNA content of recombinant cell-lines is compared with the CHO-dhfr⁻ host cell-line by FACS analysis.

**Materials**

|  |  |
|---|---|
| Washing-Buffer: | 0.1 M Tris-HCl, pH 7.4, 2 mM $MgCl_2$, 0.1% Triton X100 |
| Staining Buffer: | 0.3 $\mu$g/ml DAPI (Hoechst) in washing buffer |

**Method**

$5 \times 70^5$ cells are washed in PBS and fixed with ice cold 70% ethanol. Afterwards the cells are washed twice with washing buffer and then incubated in staining buffer for 30 minutes at RT. The DNA content is measured with the FACS Vantage (Becton and Dickinson) at 359 nm excitation and 450 nm emission.

_In-vitro_ specificity test

The human erythroleukemic cell-line TF-1 (German Collection of Microorganisms and Cell Cultures) is growth-dependent on IL3 or hGM-CSF. These cytokines display synergistic effects on proliferation, while Epo can maintain the viability for some time. The cells are routinely grown in GM-CSF and Epo containing cultivation medium.

**Test Supplements**

|  |  |
|---|---|
| Cultivation medium: | RPMI 1640, supplemented with 4mM Gln, 10% FCS, 20$\mu$g/ml transferrin, 10$\mu$M beta-mercapto-ethanol, 12ng/ml rhGM-CSF, 3U/ml rh Epo, |
| Test medium: | RPMI 1640, supplemented with 4mM Gln, 100$\mu$g/ml transferrin, 2mg/ml BSA |

**Methods**

The functionality test is performed as a MTT viability test in 96-well plates (Hammerling et al., 1996, J Pharm Biomed Anal 14(11):1455-69). Samples are diluted 1:2 fold eight times, starting with 100 ng Epo per ml in test medium. 50 $\mu$l of each sample dilution, standard dilution or blank are transferred to the 96-well testing plate. TF-1 cells are washed three times with cold PBS and adapted to $2 \times 10^5$ cells per ml in test medium. Each well of 96-well test plate is overlayed with 50 $\mu$l of the cell suspension and these cells are left for 72 hours in the $CO_2$ incubator. Afterwards 10 $\mu$l MTT solution (6 mg/ml in PBS) are added and incubated at 37°C for 4 hours. The dye is dissolved with 100 $\mu$l SDS/HCl (10% SDS in 0.1 M HCl) for another 4 hours in the dark and the Epo dependent viability is photomerically determined at 550/690 nm.

**Example 1 - Construction of plasmid Epo/neo**

**1. Construction of p2-neo**

**1.1 Preparation of the vector fragment from pSV2neo containing the SV40 early promoter**

The basis of the vector construction is the pBR322 plasmid backbone contained in pSV2neo. The smaller _Eco_RI - _Pvu_II restriction fragment includes this pBR322 backbone and the neighboring _Pvu_II - _Hind_III fragment from SV40 bears the relevant fragment of the SV40 early promoter.

Plasmid pSV2neo (ATCC 37149) is cut with the restriction enzymes _Eco_RI and _Hind_III. The two resulting fragments has a sizes of 3092 bp and 2637 bp. The 2637 bp fragment consists of an _Eco_RI - _Pvu_II restriction fragment including a pBR322 backbone and a neighboring _Pvu_II - _Hind_III fragment which contains a fragment of the SV40 early promoter. The 2637 bp is prepared and purified via gel electrophoresis.

**1.2 Preparation of the neomycin resistance gene**

The neo gene is taken from the transposon Tn5 of pSV2neo. It is amplified as a fragment containing solely the coding region of the gene. As part of the cloning strategy, recognition sites for restriction endonucleases are introduced at both ends. A *Hind*III site is built in the upstream amplification primer, an *Eco*RI and a *Spe*I site in the downstream primer. The amplified region corresponds to nucleotides 2846 to 1938 in the sequence of pSV2neo (Genbank Accession No. U02434). The oligonucleotides are designed as follows:

*Oligo 2004-01:* length: 38mer
5'- ggg gga agc ttg ttg gga agc cct gca aag taa act gg - 3' SEQ ID No.1
*5' Hind*III*:* aagctt - **g** (= pos. 2846 in pSV2neo) ttgggaagccctg............. SEQ ID No. 2

*Oligo 2004-02:* length: 42mer
5'- ggg gaa ttc act agt gag tcc cgc tca gaa gaa ctc gtc aag - 3' SEQ ID No. 3
5' *Eco*RI/*Spe*I:

gaa ttc actagt - g (= pos. 1938 in pSV2neo) agtcccgctcagaa.........SEQ ID No. 4

The amplification product of primers 2004-01 and 2004-02 is prepared by PCR using *Pwo* polymerase (Roche Diagnostics). The parameters for the process are: 20 ng of pSV2neo, 10 pmol of each primer, 10 mmol dNTPs, 2.5 U Pwo polymerase in the supplied buffer to a total volume of 50 µl; temperature profile: 5 min 95 °C, 35 times (30 sec 95 °C, 20 sec 65 °C, 90 sec 72 °C), 3 min 72 °C, cooling at 4 °C until further use.
The resulting DNA fragment of 935 bp is purified by DNA isolation columns (Mini, Wizard Promega GmbH), digested with *Eco*RI and *Hind*III, and purified via an agarose gel and eluted using Spin Columns (Supelco).

**1.3 Construction of p1-neo**

The amplified *Eco*RI-*Hind*III neo gene fragment is ligated to the *Eco*RI-*Hind*III vector fragment from pSV2-neo using Ligation Express (Clontech) and transformed into an *E.coli* host (*E.coli* SURE (Stratagene)). Transformants are selected by growth on LB medium supplemented with 50 mg/l ampicillin.
Plasmid DNA is isolated from clones and checked by restriction analysis using *Eco*RI plus *Nco*I (3 fragments of 2527 bp, 780 bp and 251 bp, respectively). Plasmid DNAs showing the expected fragments are further checked by sequencing relevant parts of the constructs. A plasmid DNA containing a verified SV40early promoter and neomycin resistance gene is designated as p1-neo.

**1.4 Preparation of the SV40 termination region SV40LTpolyA/IVS**

PCR primers are designed to amplify a fragment (nucleotides 751 to 1598) of the SV40 termination region present in pSV2neo. The upstream primer also contains a restriction site for *Spe*I. In addition to the *Bam*HI site already included at position 751 of pSV2-neo an *Eco*RI site is introduced into the downstream primer separated by a 6 nucleotide spacing region from *Bam*HI. The sequences of the two primers are as follows:

*Oligo 2004_05*: length: 40mer
5'- ggg gac tag ttt gtg aag gaa cct tac ttc tgt ggt gtg a - 3' SEQ ID No. 5
5' *Spe*I: actag - **t** (= pos. 1598 in pSV2neo) ttgtgaagga............. SEQ ID No. 6

*Oligo 2004-06:* length: 46mer
5'- ggg gga att cgg agg ggg atc cag aca tga taa gat aca ttg atg a - 3' SEQ ID No. 7
5' *Eco*RI/*Bam*HI: gaattc - **g** (= pos. 751 in pSV2neo)gatcc agacatgataag.....SEQ ID No. 8

The amplification product of primers 2004-05 + 2004-06 is prepared by PCR using *Pwo* polymerase (Roche Diagnostics) as described above. The resulting DNA fragment of 873 bp is purified using DNA isolation columns, digested with *Eco*RI and Spel and gel-purified.

**1.5 Preparation of p2-neo**

p1-neo plasmid DNA is digested using *Eco*RI + *Spe*I. The resulting linearized fragment is purified, ligated with the amplified fragment containing SV40LTpolyA/IVS and transformed into an *E.coli* host. Transformants are selected by growth on LB medium supplemented with 50 mg/l ampicillin.

Plasmid DNA is isolated from clones and checked by restriction analysis using *Eco*RI (1 fragment of 4411 bp) and *Nco*I (2 fragments of size 3631 bp and 780 bp) and *Sph*I (3 fragments of size 3499 bp, 840 bp and 72 bp). Plasmid DNAs showing the expected fragments are further checked by sequencing relevant parts of the constructs. A plasmid DNA containing a verified SV40LTpolyA/IVS is designated as p2-neo.

## 2. Construction of plasmid p3

### 2.1 Preparation of the SV40 early promoter fragment

Plasmid pSV2neo is used as a source for the SV40 early promoter fragment. The fragment size is almost identical to the one used in constructing the p2 plasmids. However the ends of the fragment are modified to introduce recognition sites for *Bam*HI and *Not*I. The oligonucleotide primers used to amplify the promoter are designed as follows:

*Oligo 2004-07:* Length: 38mer
5'- ggg ggg atc ctg tgg aat gtg tgt cag tta ggg tgt gg - 3' SEQ ID No. 9
5' *Bam*HI: gaatcc - **t** (= pos. 3435 in pSV2neo) gtggaat............. SEQ ID No.10

*Oligo 2004-08:* Length: 46mer
5'- ggg ggc ggc cgc agc ttt ttg caa aag cct agg cct cca aaa aag c - 3' SEQ ID No. 11
5' *Not*I: gcggccgc - **a** (= pos. 3093 in pSV2neo) gctttttgcaaaag............... SEQ ID No. 12

The amplification product of primers 2004-07 + 2004-08 is prepared by PCR using *Pwo* polymerase (Roche Diagnostics), as described above. The resulting DNA fragment of 365 bp is purified using DNA isolation columns, digested with *Bam*HI and *Not*I, and gel-purified.

### 2.2 Preparation of the pBluescript vector part

pBluescript II SK+ DNA is sequentially restricted using *Bam*HI and *Not*I, respectively. The DNA is dephosphorylated using alkaline phosphatase. The *Bam*HI/*Not*I fragment is purified from the small fragment via agarose gel electrophoresis prior to ligation.

### 2.3 Preparation and verification of plasmid p3

The amplified *Bam*HI-*Not*I fragment containing the SV40 early promoter is ligated into the prepared pBluescript II SK+ vector using T4 DNA Ligase (Promega GmbH). Plasmid DNA from *E.coli* SURE (Stratagene) transformants is isolated and purified from colonies on LB medium supplemented with 100 mg/l ampicillin.

Resulting DNAs are checked by restriction analysis using *Eco*RI plus *Nco*I (2 fragments of size 3039 bp and 253 bp).

Two plasmid DNAs showing the expected fragments are further checked by sequencing. Both strands of the SV40 early promoter are sequenced so that each position could be verified. The plasmid is designated as p3.

## 3. Isolation of human Epo cDNA

### 3.1 Isolation of total RNA with TRIZol® Reagent

TRIzol® reagent, used for isolation of Epo RNA from human kidney tissues (obtained from the Lainzer Krankenhaus hospital) is a mono-phasic solution of phenol and guanidine isothiocyanate. During cell lysis guanidine isothiocyanate forms a water-soluble complex with RNA while cells are disrupted. Addition of chloroform, followed by centrifugation, separates the solution into an aqueous, RNA containing and an organic phase. After separation of the aqueous phase the RNA is precipitated with isopropyl alcohol, washed with ethanol, air-dried and resuspended in RNAse free water.

Human kidney tissue fragments are cut into small pieces, forced through a 100 μm cell strainer, centrifuged (179xg/10 min) and the resulting pellet is washed three times with PBS. Then the pellet is resuspended in PBS, aliqoted in sterile tubes, frozen to -196°C and stored at -80°C until further use.

The frozen tissue is lysed by addition of 1 ml TRIzol® reagent, homogenized and incubated at 15-30°C for 5 minutes to ensure complete dissociation. After addition of 200 μl chloroform, shaking the tube and incubation for 2-3 minutes at 15-30°C the tube is centrifuged at 12000xg for 10 minutes. Following centrifugation, the upper aqueous phase is carefully transferred to a fresh tube mixed with 500 μl isopropyl alcohol and incubated at 15-30°C for 10 min. The precipitated RNA is centrifuged (12000xg, 10 min), the pellet washed with ethanol, centrifuged again, airdried and dissolved in RNAse free DEPC water. Total RNA content is measured photometrically at 260 nm.

$$1\ OD_{260nm} = 40\ \mu g\ RNA/ml$$

By evaluating the ratio of $OD_{260nm}$ and $OD_{280nm}$ (maximum absorbance of proteins) one can estimate the purity of the RNA isolation. It should range between 1.6 and 1.8.

### 3.2 mRNA isolation with Dynabeads Oligo (dT)25

Dynabeads Oligo $(dT)_{25}$ mRNA DIRECT kit employs hybridization of the polyadenosine tail RNA of eukaryotic mRNA to supermagnetic, polystyrene particles containing 25 nucleotide long chains of deoxy-thymidylate covalently attached to their surface. mRNA bound to the magnetic beads can be separated using a Dynal magnetic particle concentrator (Dynal MPC®).

| | |
|---|---|
| *Washing buffer* | Tris-HCl 10 mM, pH 8,0; LiCl 0,15 mM; EDTA 1mM |
| *2 x Binding buffer* | Tris-HCl 20 mM, pH 7,5; LiCl 1 mM; EDTA 2 mM |

For 10 $\mu$g of total RNA, 100 $\mu$l Dynabeads oligo $(dT)_{25}$ are separated in the Dynal MPC® and washed twice with 2x washing buffer. Meanwhile total RNA is adjusted to a volume of 200 $\mu$l with 1 x washing buffer and denatured by incubation at 65°C for 4 minutes. Then the RNA is mixed with the beads, incubated at room temperature for 5 minutes and separated in the Dynal MPC®. The beads are washed twice with 1x washing buffer. The polyadenylated RNA is eluted from the Dynalbeads Oligo $(dT)_{25}$ by incubation with elution buffer (2 x 10 $\mu$l) for 4 minutes at 65°C. Dynabeads are separated in the Dynal MPC® and the supernatant is immediately transferred to a new RNAse free microcentrifuge tube. The eluate is used directly for reverse transcription.

### 3.3 Reverse Transcription

The specific primer for Epo is denatured by 4 min incubation at 80°C and the mRNA is denatured by 5 min incubation at 65°C. The following components are added to a sterile 1.5 ml microcentrifuge tube on ice:

| Reagent | Final concentration |
|---|---|
| mRNA | 10 $\mu$l |
| MMLV (200 U/$\mu$l) | 0,25 $\mu$l |
| Boehringer PCR buffer (10 x) | 2 $\mu$l |
| dNTPs (10 mM) | 2 $\mu$l |
| $MgCl_2$ (50 mM) | 1 $\mu$l |
| Epo for (100 pmol/$\mu$l) | 1 $\mu$l |
| DTT (0,1 M) | 0,25 $\mu$l |
| RNAse inhibitor (40 U/$\mu$l) | 0,25 $\mu$l |
| $H_2O$ | 3,25 $\mu$l |

| | | |
|---|---|---|
| Incubation: | 60 min | 37 °C |
| Inactivation: | 5 min | 100 °C |

### 3.4 Polymerase chain reaction

The following components are added to a sterile 1.5 ml microcentrifuge tube at 4°C. PCR conditions are listed below.

| Reagent | Epo |
|---|---|
| Template | cDNA Epo 5 $\mu$l |
| polymerase | Vent 1 U (0,5$\mu$l) |
| polymerase buffer (10x) | Vent buffer 1 x (10$\mu$l) |
| dNTPs (10 mM) | 200 $\mu$M (2$\mu$l) |
| $MgCl_2$ (50 mM) | / |
| Primer for (10 pM) | 30pM (3$\mu$l) |
| Primer back (10 pM) | 30 pM (3$\mu$l) |
| DMSO | / |
| $H_2O$ | 76,5 $\mu$l |

| PCR cycle | | |
|---|---|---|
| 1 Denaturation | 95°C | 2 min |
| 2 Denaturation | 94°C | 45 sec |
| 3 Primer annealing | 58°C | 30 sec |
| 4 extension | 72°C | 1 min |
| 5. finish extension | 72°C | 10 min |
| 6. cycles | 30 | |

The PCR amplification products are analyzed by agarose gel electrophoresis.

### 3.5 *Agarose gel electrophoresis*

| | |
|---|---|
| *6 x BX buffer* | bromphenol blue 0,25 %; xylene cyanol 0,25 %, glycerol 30 % |
| *TAE buffer* | Tris base 242g; glacial acetic acid 57,1 ml; EDTA (0,5 M, pH 8,0) 100 ml; adjusted to 1000 ml $H_2O$ |
| *Lambda-Marker III* | 10 $\mu$g bacteriophage Lambda-wildtype-Dann (2,5 $\mu$l Hind III + 2,5 $\mu$l Eco R I + 20 $\mu$l buffer R (Fermentas) filled up with $H_2O$ ad 200 $\mu$l; |
| | 1 h at 37°C digested, 20 min at 65 C inactivated; supplemented with 40 $\mu$l BX-loading buffer) |

1 g agarose and 99g 1xTAE buffer are melted in the microwave oven, cooled down to approximately 60°C and supplemented with 3 $\mu$l of ethidium bromide stock solution (10 mg/ml). Gels are run in 1xTAE buffer at 100-300 V for approximately 30 min, depending on the length of the DNA fragments to be separated. Each lane contains 10 $\mu$l sample mixed with 2 $\mu$l 6 x BX buffer. Identification of DNA fragments is based on comparison with a Lamba/Hind III digest molecular weight standard.

### 3.6 Preparation of PCR products and vectors for ligation

*3.6.1 Restriction of Vector DNA and insert for sticky end cloning*

10u of restriction enzyme and appropriate restriction buffers are mixed with 1$\mu$g vector DNA and insert according to manufacturers instructions. The mixture is incubated at 37°C (30°C for Smal) between 30 and 60 min, depending on the enzymes used, vector and insert. Then the enzyme is inactivated by heating up to 65°C for 10 min and the reaction mixture is analyzed by agarose gel electrophoresis.

*3.6.2 Ligation*

*pIRESneoSV40 vector*

pIRES*neo* vector (Clontech laboratories) contains the internal ribosome entry site (IRES) of the encephalomyocarditis virus (ECMV), which permits the translation of two open reading frames from one messenger RNA. The expression cassette of pIRES*neo* contains the human cytomegalovirus (CMV) major immediate early promoter/enhancer followed by a multiple cloning site (MCS), the ECMV IRES followed by the neomycin phosphotransferase gene and the polyadenylation signal of the bovine growth hormone. In this vector the CMV promoter is replaced by the SV40 early promoter.

Vector and PCR product are ligated with T4 DNA ligase. For optimal ligation approximately 20 ng vector and 200 ng insert (depending on the length) are used in a molar ratio of about 1:10 and mixed with following reagents in a total volume of 10 $\mu$l H$_2$O. The incubation is performed overnight at 15°C and 3 h at RT. Then the ligase is heat-inactivated by incubation at 65°C for 10 minutes.

| Reagent | Final amount |
| --- | --- |
| Vector (pIRESneoSV40) | ~ 20 ng |
| Insert (Epo) | ~ 200 ng |
| T4 DNA Ligase | 1U (1 $\mu$l) |
| Buffer (5x) | 1x (2 $\mu$l) |
| H$_2$O | ad 10 $\mu$l |

*3.6.3 Bacteria and culture media*

| | |
| --- | --- |
| *JM109* | (Promega, USA) |
| *LB-medium* | peptone from casein 10 g; yeast extract 5 g; NaCl 10 g, adjusted to 1000 ml with H$_2$O and set to pH 7,0 with 5M NaOH |
| *LB agar* | 15 g agar in 1000 ml LB-medium |
| *LB-Amp* | 100 $\mu$l ampicillin (100 mg/ml) in 1000 ml LB-medium |
| *SOC medium* | bacto tryptone 20 g; yeast extract 5 g; NaCl 10 mM; KCl 3 mM; MgCl$_2$ 10 mM; glucose 20 mM, MgSO$_4$ 10 mM |

*3.6.4 Transformation using CaCl$_2$*

Preparation of competent bacteria (JM109)

10 ml of LB medium is inoculated with *E.coli* (JM109) and grown overnight at 37°C. 4 ml bacterial culture is diluted 1:100 in LB medium and grown until having reached OD$_{260\,nm}$ of 0.8. Bacteria are centrifuged at 4500 rpm for 10 min at 4°C and the cell pellet is resuspended in 10 ml 0.1 M CaCl$_2$ (4°C)/50 ml bacterial suspension used. The cells are centrifuged, the pellet is resuspended in 2 ml 0.1 M CaCl$_2$ and aliquoted to a total volume of 100 $\mu$l, frozen in liquid nitrogen and stored at -80°C.

Transformation

In pre-chilled 17x100 mm polypropylene culture tubes 5-10 ng plasmid DNA is added to JM109 competent bacteria, gently mixed and put on ice for 30 min. Then the cells are heat-shocked for 45 seconds in a waterbath at exactly 42°C without shaking and immediately placed on ice for 2 minutes. Then 900 $\mu$l SOC medium is added to the tube and incubated for 30 min at 37°C before plating 100 $\mu$l of bacteria suspension on LB-Amp plates.

*3.6.5 Screening and establishing glycerin cultures*

Ampicillin resistant colonies are screened for the inserted DNA fragment by PCR technique. Portions of ampicillin resistant colonies are mixed with the PCR reaction mixture and with specific primers against the cloned DNA fragment (see below). Positive colonies show PCR-amplified DNA bands in agarose gel electrophoresis. These colonies are then propagated in LB-Amp medium for further analysis and plasmid purification. For further use and storage 1 ml of desired bacteria culture is mixed with 500 $\mu$l glycerin (87 %) and stored at -80°C.

*3.6.6 Wizard® Plus SV Minipreps DNA Purification System*

| | |
|---|---|
| *Cell resuspension solution* | Tris-HCl 50 mM, pH7.5; EDTA 10 mM, RNase A 100 μg/ml |
| *Cell lysis solution* | NaOH 0.2 M, SDS 1 % |
| *Neutralization solution* | guanidine hydrochloride 4.09M, potassium acetate 0.759M; glacial acetic acid 2.12M, pH 4.2 |
| *Column wash solution* | potassium acetate 60 mM, Tris-HCl 10 mM, pH 7.5; ethanol 60% |

2-3 ml LB-Amp medium are inoculated with a single colony and incubated at 37°C over night. The solution is centrifuged (12000xg, 5 min) and the resulting pellet is thoroughly resuspended in 250 μl resuspension solution and then 250 μl of cell lysis solution, mixed by inverting the tubes 4 times and incubated at RT for 1-5 min. Thereafter 10 *l of alkaline protease solution (incubated at RT for 5 min) and 350 μl neutralization solution ware added. The tube is immediately mixed by inverting it 4 times and the bacterial lysate is centrifuged at 12000xg for 10 min at RT. The bearded lysate is transferred to Spin Columns and centrifuged (12000xg, 5 min) and the column is washed twice with washing solution (750 μl/250 μl). The DNA is eluted with 100 μl nuclease-free water.

*3.6.7 Sequencing of plasmids*

The inserted sequences are sequenced by IBL (Gerasdorf, Austria) and by GenXpress (Maria Wörth, Austria) with specific primers. Oligonucleotide primers for the amplification of Epo and SV40early promoter and for sequence analysis are listed below.

| Oligonucleotide primer | Sequence | |
|---|---|---|
| *SV40early promoter* | | |
| SV40 early ClaI for | 5'-aga tcg atc aag ctt ttt gca aaa gcc tag-3' | SEQ ID No. 13 |
| SV40 early NruI back | 5'-agt cgc gag cgc agc acc atg gcc tg-3' | SEQ ID No.14 |
| SV40 early 281 back | 5'-gcc cag ttc cgc cca ttc-3' | SEQ ID No. 15 |
| *Epo* | | |
| Epo BamHI for | 5'-tag gat cct cat ctg tcc cct gtc ctg c-3' | SEQ ID No.16 |
| Epo EcoRI back | 5'-tag aat tcc gcc atg ggg gtg cac gaa tgt cc-3' | SEQ ID No. 17 |
| Epo 221 for | 5'-taa ctt tgg tgt ctg gga-3' | SEQ ID No.18 |
| Epo 204 back | 5'-tcc cag aca cca aag tt-3' | SEQ ID No.19 |
| *pIRESneo* | | |
| pIRESneo 181 back | 5'-tta ggg tta ggc gtt ttg cg-3' | SEQ ID No.20 |
| piRESneo 1016 for | 5'-act cac ccc aac agc cg-3' | SEQ ID No. 21 |
| pIRESneo 2786 for | 5'-ggcc aaa caa cag atg gct-3' | SEQ ID No. 22 |
| pIRES-200 back | 5'-tgg aaa gag tca aat ggc-3' | SEQ ID No. 23 |

## 4. Construction of plasmid p5

### 4.1 Preparation of the Epo gene fragment

The structural gene for Epo (human erythropoietin) is amplified by PCR using pSVGPIRNEO as a template DNA. The sequence of Epo is given in GenBank Accession No. M 11319.1. Recognition sites for *Not*I and *Ksp*I are introduced into the upstream and downstream primer, respectively. The primers are designed as follows:

*Oligo 2004-09:* Length: 45mer
5'- ggg ggc ggc cgc atg ggg gtg cac gaa tgt cct gcc tgg ctg tgg - 3' SEQ ID No. 24
5' *Not*I: acaaccgc a(= pos. 665 in PSVGPIRNEO) tgggggtg............... SEQ ID No. 25

*Oligo 2004-10:* Length: 44mer
5'- ggg gcc gcg gtc atc tgt ccc ctg tcc tgc agg cct ccc ctg tg - 3' SEQ ID No.26
5' *Ksp*I: ccgcgg - **t** (= pos. 1246 in PSVGPIRNEO) catctgtcccct.............. SEQ ID No. 27

The amplification product of primers 2004-09 + 2004-10 is prepared by PCR using *Pwo* polymerase (Roche Diagnostics), as described above. The resulting DNA fragment of 604 bp is purified using DNA isolation columns, digested with *Ksp*I and *Not*I, and gel-purified. The resulting 592 bp *Ksp*I/*Not*I Fragment is used in the triple ligation described below.

### 4.2 Preparation of the termination region SV40LTpolyA/IVS

The termination region of SV40LTpolyA/IVS is recloned from pSV2neo by PCR in a similar manner to that described above in section 1.4 for the construction of p2-neo except that the primers are designed with different restriction endonuclease recognition sites: the site for *Ksp*I (=*Sac*II) is included into the upstream primer and the sites for *Sac*I and *Eco*RI into the downstream primer.

> *Oligo 2004-11:* length: 42mer
> 5'- ggg gcc gcg gtt tgt gaa gga acc tta ctt ctg tgg tgt gac - 3' SEQ ID No.28
> *5' Ksp*I: ccgcgg - t (= pos. 1598 in pSV2neo) ttgtgaaggaa............. SEQ ID No.29

> *Oligo 2004-12:* length: 46mer
> 5'- ggg gga gct cga att cga tcc aga cat gat aag ata cat tga g - 3' SEQ ID No. 30
> *5' Sac*I/*Eco*RI*:* gagctc gaattc - **g** (= pos. **752** in pSV2neo) atccagacatg.....SEQ ID No. 31

The amplification product of primers 2004-11 + 2004-12 is prepared by PCR using *Pwo* polymerase (Roche Diagnostics), as described above. The resulting DNA fragment of 873 bp is purified using DNA isolation columns and digested with *Ksp*I and *Sac*I. The resulting DNA fragment of 858 bp is then gel-purified.

### 4.3 Preparation of the p3 vector part

p3 plasmid DNA is sequentially digested using *Not*I and Sad, respectively. The DNA is treated with alkaline phosphatase and the vector fragment is gel-purified.

### 4.4 Triple ligation and isolation of plasmid p5

The *Not*I/*Sac*I vector part of plasmid p3, the *Ksp*I/*Not*I Epo gene and the *Ksp*I/*Sac*I termination region SV40LTpolyA/IVS are ligated in one ligation reaction (Ligation Express, Clontech). Transformants of are selected on LB medium supplemented with 100 mg/l ampicillin.
Positive transformants containing both fragments inserted are screened by colony hybridization using both amplified fragments 2004-0912004-10 and 2004-11/2004-12, as labeled probes. Ten clones which gave a positive hybridization signal with both probes are chosen for a "midi" scale plasmid preparation (Qiagen).
Restriction analysis is performed using the enzymes *Bam*HI (1 fragment 4723 bp), *Eco*RI (2 fragments, 2913, 1810 bp) and *Pvu*II (4 fragments 2513, 1204, 903, 103 bp). Two clones showing the correct restriction fragments are selected and checked by sequencing. The whole cassette cloned into pBluescript II SK+ is sequenced and compared to the expected nucleotide sequence. Every single nucleotide could be successfully verified. The plasmids are designated p5.

### 5. Construction of pEpo/neo

### 5.1 Construction of pEpo/neo 12-1

p5 plasmid DNA is digested with *Bam*HI and *Eco*RI and the resulting 1792 bp fragment representing the cassette of SV40promoter-Epogene-SV40terminator is gel-purified.
Plasmid p2-neo is also digested with *Bam*HI and *Eco*RI and the linearized vector gel-purified. Additionally the DNA is dephosphorylated using alkaline phosphatase and purified with Amicon Micropure enzyme removers.
Both fragments, the 4411 bp p2-neo vector and the 1792 bp cassette from p5, are ligated (Ligation Express, Clontech) and transformed into *E.coli* SURE. Plasmid DNA is isolated from various transformants grown on LB medium supplemented with 70 mg/l ampicillin and analyzed by digestion using restriction endonucleases *Pvu*II, *Eco*RI and *Nco*I.
A clone showing the expected fragments (*Eco*RI: 6191 bp, *Nco*I: 4085, 1326 and 780 bp, *Pvu*II*:* 3273, 2130, 685 and 103 bp) is selected and designated as pEpo/ neo-12.
For additional purification the DNA is retransformed into *E.coli* SURE (see above) and plasmid DNA prepared using a "Midi-prep" procedure (Qiagen) from a culture inoculated by a single colony (pEpo/neo-12-1). Restriction analysis is performed using the following enzymes: BamHI, *Hind*III, *Eco*RI, *Nco*I, *Not*I, *Pst*I, *Spe*I, *Sph*I, *Pvu*II*, Nar*I. The expected fragments and sizes could be found, verifying the clone as a correct pEpo/neo clone.

**5.2 The final construction of pEpo/neo**

The upstream region of the Epo gene in pEpo/neo-12-1 is changed at position minus-3 from the start ATG. An additional nucleotide A is introduced to result in the purine base G at position -3 from start ATG. A purine at that position may improve the expression level of the gene. For that purpose the Epo gene is reamplified using an adapted upstream primer 2004-09-a:

> *Oligo* 2004-09-a: length: 46mer
> 5'- gggggcggccgcaatgggggtgcacgaatgtcctgcctggctgtgg - 3' SEQ ID No. 32

The amplification product of primers 2004-09_a + 2004-10 is prepared by PCR using *Pwo* polymerase (Roche Diagnostics), as described above. The resulting DNA fragment of 605 bp is purified using DNA isolation columns and digested using *Ksp*I and *Not*I. The resulting DNA fragment of 593 bp is then gel-purified.

pEpo/neo-12-1 plasmid DNA is digested with *Ksp*I and *Not*I, respectively, to remove the Epo gene. The 5599 bp fragment is then gel-purified. Both prepared DNAs are ligated to each other (Ligation Express, Clontech). Plasmid DNA from transformants is isolated and purified from colonies on LB medium supplemented with 70 mg/l ampicillin. DNAs are analyzed by restriction using *Nco*I in a first screening.

A positive clone is selected to isolate DNA using a "Midi prep" procedure (Qiagen). An extended restriction analysis is performed using *Bam*HI, *Hind*III, *Eco*RI, *Nco*I, *Not*I, *Pst*I, *Spe*I, *Sph*I, *Pvu*II, *Nar*I. The expected fragments and sizes could be found, verifying the clone as a correct pEpo/neo. Every single nucleotide of the whole cassette (SV40early_promoter - **neo gene -** SV40LTpolyA/IVS - SV40early_promoter - **Epo gene -** SV40LTpolyA/IVS) inserted in the pBR322 vector-part is also confirmed by sequencing.

**Example 2 - Construction of plasmid Epo/dhfr**

**1. Construction of p2-dhfr-CDS**

**1.1 Preparation of the dhfr gene**

The dhfr gene used for the vector construction is taken from a mouse cDNA, present in plasmid pLTRdhfr26 (ATCC 37295). The nucleotide sequence of the mouse dhfr cDNA (MUSDHFR) is available as GenBank Accession No. L26316.

The dhfr is amplified from pLTRdhfr26 using primers designed to produce a fragment containing the coding region from the start ATG at position 56 to the stop codon TAA at position 619. As for the amplification of the neomycin resistance gene described above, *Hind*III and *Spe*I sites are introduced in the upstream and downstream amplification primers, respectively. An *Eco*RI site is also introduced into the reverse primer beside the Spel site. The sequence of the oligonucleotides is as follows:

> *Oligo 2004-13:* length: 39mer
> 5'- ggg <u>gaa act t</u>at ggt tcg acc att gaa ctg cat cgt cgc - 3' SEQ ID No. 33
> 5' *Hind*III: <u>aagctt</u> - A (= pos. 56 in MUSDHFR) TGgttcgaccattg............. SEQ ID No. 34

> *Oligo 2004-14:* length: 42mer
> 5'- ggg <u>gaa ttc act agt</u> tag tct ttc ttc tcg tag act tca aac - 3' SEQ ID No. 35
> 5' *Eco*RI <u>Spe</u>I:

>> <u>gaattc actag</u> - **t** (= pos. 619 in MUSDHFR) tagtctttcttctcgtagacttcaaact..... SEQ ID No. 36

The amplification product of primers 2004-13 + 2004-14 is prepared by PCR using *Pwo* polymerase (Roche Diagnostics), as described above. The resulting DNA fragment of 588 bp is purified using DNA isolation columns, digested with *Hind*III and *Eco*RI and gel-purified.

**1.2 Preparation of p7-dhfr-CDS**

The amplified *Eco*RI-*Hind*III dhfr gene fragment is ligated to the *Eco*RI-*Hind*III vector fragment from pSV2-neo using Ligation Express (Clontech), and transformed into an *E.coli* host. Transformants are selected by growth on LB medium supplemented with 50 mg/l ampicillin. Plasmid DNA from transformants is isolated and purified from colonies on LB medium supplemented with 50 mg/l ampicillin.

Plasmid DNA is isolated from clones and checked by restriction analysis using *Eco*RI plus *Sca*I (3 fragments of size 2225 bp, 514 bp and 473 bp).

Plasmid DNAs showing the expected fragments are further checked by sequencing relevant parts of the constructs. A plasmid DNA containing a verified SV40early promoter and dihydrofolate reductase gene is designated as p1-dhfr-CDS. The analysis of the sequences revealed one deviation within the dhfr gene from the sequence published in MUSDHFR, specifically a change from T to C at position 451 of the MUSDHFR sequence. Subsequent sequencing showed that this change is also present in the source plasmid. However the resulting change does not cause a change in the amino acid sequence encoded by nucleotide sequence since CTT and CTC both encode leucine.

## 1.3 Preparation of p2-dhfr-CDS

p1-dhfr-CDS plasmid DNA is digested using *Eco*RI + *Spe*I. The resulting linearized fragment is purified and ligated with the amplified fragment containing SV40LTpolyA/IVS (described above). Following transformation and selection, resulting plasmids are analyzed by restriction analysis using *Acc*I (3 fragments of 2994, 855 and 216 bp). A few are selected and additionally analyzed using *Hinc*II (2 fragments of 3466 bp and 599 bp, respectively), *Afl*III (2 fragments of 2872 bp and 1193 bp, respectively) and *Bgl*I (2 fragments of 2371 bp and 1694 bp, respectively).

A plasmid DNA showing all the expected fragments in the correct sizes is further checked by sequencing. A verified plasmid is designated as p2-dhfr-CDS.

## 2. Construction of pEpo/dhfr

### 2.1 Preparation of pEpo/dhfr 21

p5 plasmid DNA is digested with *Bam*HI and *Eco*RI and the resulting 1792 bp fragment representing the cassette of SV40promoter-Epogene-SV40terminator is gel-purified.

Plasmid p2-dhfr-CDS is also digested with *Bam*HI and *Eco*RI and the linearized vector is gel purified and eluted using Supelco spin columns. Additionally the DNA is dephosphorylated using alkaline phosphatase and purified with Amicon Micropure enzyme removers.

Both fragments, the 4053 bp p2-dhfr-CDS vector and the 1792 bp cassette from p5, are ligated (Ligation Express, Clontech) and transformed into *E.coli* SURE. Transformants colonies grown on LB medium supplemented with 70 mg/l ampicillin are hybridized using Epo gene (PCR-product) as a probe. Plasmid DNA is isolated from various positive clones and analyzed by digestion using restriction endonuclease *Nco*I.

A clone showing the expected fragments (*Nco*I: 4085 bp and 1760 bp) is selected and designated as pEpo/dhfr-21. For additional purification the DNA is retransformed into *E.coli* SURE (see above) and plasmid DNA prepared using a "Midi-prep" procedure (Qiagen) from a culture inoculated by a single colony (pEpo/dhfr-21-1).

Restriction analysis is performed using the following enzymes: *Bam*HI, *Hind*III, *Eco*RI, *Nco*I, *Not*I, *Pst*I, *Spe*I, *Sph*I, *Pvu*II, *Nar*I. All the expected fragments and sizes could be found, verifying the clone as a correct pEpo/dhfr-21.

### 2.2 The final construction of pEpo/dhfr

In the same way as for pEpo/neo the upstream region of the Epo gene in Epo/dhfr-21 is changed at position -3 referred to the start ATG. An additional nucleotide A is introduced to result in the purine base G at position -3 from start ATG. The Epo gene is reamplified as described in Example 1, section 4.2.

pEpo/dhfr-21 plasmid DNA is digested with *Ksp*I and *Not*I, to remove the Epo gene. The 5259 bp fragment is then gel-purified.

Both prepared DNAs are ligated to each other (Ligation Express, Clontech). Plasmid DNA from transformants is isolated and purified from colonies on LB medium supplemented with 70 mg/l ampicillin. DNAs are analyzed by restriction using *Nco*I in a first screening.

A positive clone is selected to isolate DNA using a "Midi prep" procedure (Qiagen). An extended restriction analysis is performed using *Bam*HI, *Hind*III, *Eco*RI, *Nco*I, *Not*I, *Pst*I, *Spe*I, *Sph*I, *Pvu*II, *Nar*I. The expected fragments and sizes could be found, verifying the clone as a correct pEpo/dhfr.

Every single nucleotide of the whole cassette (SV40early promoter - **dhfr gene -** SV40LTpolyA/IVS - SV40early promoter - **Epo gene -** SV40LTpolyA/IVS) inserted in the pBR322 vector-part is also confirmed by sequencing.

## Example 3 - Recombinant CHO-cells generated from pEpo/neo and pEpo/dhfr

$1-5 \times 10^4$ cells per $cm^2$ are seeded in $25 cm^2$ T-flask bottles or 96-well plates the day before the lipofectin transfection is performed. The two plasmids are mixed at the ratio of 50:1 = Epo/neo:Epo/dhfr and allowed to adsorb to the lipofectin reagent (GIBCO/BRL) according to the manufacturer's protocol.

In brief, we used 0.25 $\mu$g DNA/cm$^2$ and 1.5 $\mu$l lipofectin-reagent/cm$^2$ and adjusted this DNA/lipid cocktail to 200 $\mu$l/cm$^2$ cell layer. Then the cells are overlaid with the transfection cocktail for four hours in serum-free DMEM, before the DNA-containing medium is replaced with cultivation medium. After cultivation for 24 hours in the serum-containing medium we switched to selection medium. Transfected cell-pools are first cultivated in selection medium to confluence and then in amplification medium (4.8x10$^{-8}$ M MTX) before screening the cell culture supernatants by ELISA for Epo production. Highest producers are determined, the MTX concentration increased two-fold and best producers used for further cultivation. 7 recombinant cell pools are selected and comparison made of the growing properties, the Epo productivity, the protein pattern (by western blot analysis), the Epo functionality and the chromosomal stability.

Transfection in T25-flasks is carried out with 2.5 $\mu$g pEpo/neo, 0.05 $\mu$g pEpo/dhfr and 15 $\mu$l lipofectin (09/T25/1 and 09/T25/2) per T25-flask and with 2 $\mu$g pEpo/neo, 0.4 $\mu$g pEpo/dhfr and 15 $\mu$l lipofectin (09/T25/3 and 09/T25/4) per T25-flask.

Additionally five plates are each transfected with 10 $\mu$g pEpo/neo, 0.2 $\mu$g pEpo/dhfr and 60 $\mu$l lipofectin per plate (09/96/1 - 09/96/5), five plates with 8 $\mu$g pEpo/neo, 1.6 $\mu$g pEpo/dhfr and 60 $\mu$l lipofectin per plate (09/96/6 - 09/96/10). Plates 11 and 12 are transfected with 6.25 $\mu$g pEpo/neo, 0.08 $\mu$g pEpo/dhfr and 37.5 $\mu$l lipofectin each.

In brief, 0.25 $\mu$g DNA/cm$^2$ and 1.5 $\mu$l lipofectin-reagent/cm$^2$ are used and this DNA/lipid cocktail adjusted to 200 $\mu$l/cm$^2$ cell layer.

The series of transfections is mainly done in microtitre plates since previous experiments show that the number of clones in one cultivation unit is maximum three to five. This means easier isolation of a monoclonal transfectant than isolation from hundreds of clones in the T-flasks. Table 1 describes the number of clones per 96-well plate and the ELISA titers with and without amplification pressure. Transfected cell pools are first cultivated in selection medium to confluence and then in amplification medium (4.8x10$^{-8}$ M MTX) before screening the cell culture supernatants by ELISA for Epo-production. Approximately 1000 growing wells are screened, and 50 such cultures tested for specific Epo-productivity with increased MTX concentration. Highest producers are determined, the MTX concentration increased two-fold and best producers used for further cultivation.

The selection and first amplification steps are done in the 96-well plate and after screening all clones, growing in 4.8x10$^{-8}$ M MTX, 7 clones are selected, designated 09/96/1 F5, 09/96/3D5, 09/96/3H5, 09/96/5D4, 09/96/5H1, 09/96/6C5 and 09/96/7E6, and their growing properties, Epo productivity, protein pattern in western blots, Epo functionality tests and chromosomal stability compared.

The cell doubling time seems to be the same for all clones and they can be split 1:2 to 1:5 twice a week. Enhancing the MTX concentration from 9.6x10$^{-8}$ M to 1.9x10$^{-7}$ M also improves the productivity, while further doubling the MTX concentration does not influence the ELISA value. So subcloning is performed at 3.8x10$^{-7}$ M MTX. Immunofluorescence is analyzed at 1.9x10$^{-7}$ M MTX where the single cultures do not differ significantly.

Cell morphology is compared by light microscopy and Coulter Counter nucleus DNA analysis. Clones 09/96/7E9, 09/96/6C5, 09/96/5H1, 09/96/5D4 and 09/96/3D5 feature the same nucleus size distribution as the host cell line CHO-DHFR$^-$. In contrast, cell lines 09/96/1 F5 and 09/96/3H5 have larger nuclei. It is known from previous experiments that this results from an extended number of chromosomes. It is therefore decided to use clone 09/96/3D5 for further stabilization.

The functionality test for Epo on TF-1 cells gives the same slope for all seven culture supernatants compared with the recombinant pharmaceutical product.

The recombinant protein is tested by SDS PAGE and western blotting at each MTX concentration and only minor changes are found in any of the recombinant culture supernatants. The clones produce Epo.

### Summary and Discussion

The selection of a recombinant, Epo expressing CHO-cell line from the construction of eukaryotic expression vectors up to the transfection of mammalian cells and isolation of polyclonal Epo expressing cell pools is described. The analytical basis is set mainly with ELISA, immunofluorescence, western blotting and *in vitro* functionality tests. All these methods are established in concentration ranges that are capable of screening low producing cell pool culture supernatants with only ng/ml amounts as well as more stabilized recombinant cells.

A recombinant CHO-pool is generated, in which the gene copy number is amplified stepwise with up to 3.8x10$^{-7}$ M MTX. These cells can be split 1:3 to 1:4 twice a week and each time elevated levels of Epo are detected in ELISA.

### Example 4 - Further selection of a recombinant cell-line

Recombinant cell-pool 09/96/3D5 is used for further stabilization. MTX concentration is increased stepwise to 0.38 $\mu$M MTX. At this amplification level recombinant 3D5 cells are subcloned with 10 and 20 cells per well. Screening of culture supernatants of wells with single clones is performed by ELISA. Table 2 shows the subcloning conditions and efficiencies of recombinant cell-pool 3D5 in the presence of 0.38 $\mu$M MTX. 300 supernatants of single clones are tested. Clones that have elevated Epo titers four days after passaging are selected with 0.77 $\mu$M MTX in 24 well plates.

Seven of these clones are conserved in liquid nitrogen and selected for further amplification of gene copy number by increasing MTX concentration to 1.54 $\mu$M.

Table 3 compiles the plating conditions and efficiencies of the second round of stabilization. Here the clones 09/96/3D5/1 H9 and 09/96/3D5/18E5, are subcloned a final time with 1.54 $\mu$M MTX. The cell counts per well are reduced to 4 cells. 260 single clones are screened of which more then twenty clones of each subcultivation are transferred to T-flasks and screened for specific productivity. The final production clones are settled by criteria such as specific expression rate, growth conditions and nucleus size distribution. Clones showing tetraploidy are discarded because of the experience that such cells tend to show complicated growth patterns in bioreactors. After screening the following six subclones (four 1 H9 and two 18E5 subclones) are chosen, which are frozen in liquid nitrogen.

| | | |
|---|---|---|
| 09/96/3D5/1H9/4C2 | 09/96/3D5/1H9/6C2 | 09/96/3D5/1H9/6D4 |
| 09/96/3D5/1H9/15B4 | 09/96/3D5/18E5/7A6 | 09/96/3D5/18E5/15C3 |

**Example 5 - Adaptation to serum-free cultivation medium**

The final six recombinant cell-lines from Example 4 are chosen for adaptation to serum-free cultivation conditions after the last subcloning step.

Cells are seeded in the 7. - 12. passage after subcloning with approximately $5\times10^4$ cells/cm$^2$ into T25-flasks and are cultivated 3-4 days to confluence. At this time point the medium is replaced completely with serum-free adaptation medium and afterwards 80% of the medium is renewed daily. All suspended cells are returned to the culture. After the adaptation time, when nearly all cells grew in suspension, the clones are passaged twice a week and cultivated as suspension-culture.

Clones are cultivated for 11 -13 passages in serum-free adaptation medium before cryopreservation. Six ampoules with $5\times10^6$ cells each are frozen of every cell-line in liquid nitrogen with serum-free freezing medium. After thawing, the clones are cultivated in serum-free production medium. Analytical characterization to select for the production clone is done with supernatants in the second or third passage after thawing.

Analytical tests included:

Specific growth rate [$\mu$] - ($\mu$ = In ($X_2/X_1$) / days)
Specific productivity [$q_P$] - ($Q_P$ = product-generation x $10^6$ / (cell counts x days))
Western blot
Isoelectric focussing
DNA content and stability
Clone stability

All six cell-lines could be grown in serum-free growth media and are split twice a week. Cryopreservation is also performed without serum and after thawing the cultivation medium is switched to serum free production medium. This formulation is enriched in glucose and amino acids.

After 5 passages different protein and cellular parameters are determined and one production clone (09/96/3D5/1 H9/6C2; abbreviated 6C2) and one back up clone (09/96/3D5/1 H9/4C2; abbreviated 4C2) selected.

**Example 6 - Comparison of the recombinant cell-lines**

Growth properties of the six cell-lines from Examples 4 and 5 are calculated over several weeks by determining the cell counts in culture as well as splitting ratios during passaging. The Epo productivity is tested by ELISA. From that data the specific productivity and specific growth rate as described above are calculated

Table 4 summarizes the data received under standard cultivation conditions with a splitting ratio of 1:3 after three days cultivation.

Cell counts (measured by Coulter Counter) after splitting and after additional three days are shown.

SDS-PAGE under reducing conditions

The supernatants of the six cell-lines are separated by SDS-PAGE and compared for differences in the molecular weight. The six supernatants indicate identical SDS patterns with a smear, commonly seen in such highly glycosylated proteins (data not shown).

The comparable commercial available product migrates as a more distinct band probably arising from separating distinct bands during down stream processing.

IEF-Western blot

The IEF-western blot analysis should reflect potential microheterogeneities of the glycoproteins. According to the amount of protein that is loaded on the gel to fourteen bands become visible. There is one characteristic double-band seen on the western blot approximately in the middle of the gel; the next band down under this double-band is defined as band number one and 9 to 10 bands are visible in this acidic part of the gel. The comparable commercial product gave four major bands that correspond to band number six to nine in the heterogeneous product.

DNA content of recombinant cells

The DNA content is proportional to the numbers of chromosomes of cell-lines. The stability of a recombinant cell-line is in part influenced by the chromosomal count and the identity of DNA content is verified by comparison to the host cell-line (CHO dhfr⁻).

**Summary and Discussion**

The isolation of recombinant, Epo expressing CHO cell-lines is described herein. After two rounds of subcloning six cell-lines are compared for different properties as the basis for the designation of one final production clone. The analytical basis is mainly ELISA, western blotting and IEF tests as well as DNA measurement by FACS analysis.

The western blot pattern of the recombinant culture supernatants shows several additional lower molecular weight bands compared to the commercial purified protein. One explanation is that these additional bands represent isoforms which are removed during the down stream processing leading to the commercial product compared. Another possibility that artificial bands are being detected due to incomplete uptake of SDS.

Isoelectric focussing gives identical isoform-distribution for all cell culture supernatants, irrespective of their Qp.

The best producing clone and easiest-handling is clone 6C2 which is chosen as production clone. As back-up clone 4C2 is chosen. Both clones can be propagated in roller bottles.

**Example 7 - Cultivation of CHO cells in T-Flasks**

Recombinant human Erythropoietin is produced in a Chinese hamster ovary cell line (CHO) under serum free conditions in T-Flasks. The culture is seeded with a $2.67 \times 10^5$ cells/mL. After a three day incubation period a final cell density of $9.35 \times 10^5$ cells/mL is reached (=> $\mu$ = 0.42 days$^{-1}$).

Examples 1 to 6 describe the preparation of a number of CHO clones which express Epo. Of the six clones obtained in Examples 4 and 5, clone CHO 6C2 is chosen due to its superior high cell specific productivity and its high specific growth rate.

**Example 8 - Cultivation of CHO cells in a bioreactor**

The CHO cell line 6C2 is cultivated in Fed-Batch (T43C6C2) mode in a 150 L bioreactor. Using a cell culture medium consisting of amino acid-supplemented 50:50 DMEM/Hams F12 and containing 0.25% of a plant peptide, 0.1% lutrol, 1.54 $\mu$M methotrexate (MTX), 4 g/L glucose, 2.5 g/L NaHCO$_3$, ethanolamine, ferric citrate, ascorbic acid and sodium selenite. The medium did not contain any expensive functional proteins (recombinant or from natural sources). Components derived from an animal origin are present. The cells are seeded at around $5 \times 10^5$ cells/mL in 56 L medium. The pO$_2$ is set to 50% air saturation, temperature to 37°C and the pH to 7.0 and kept constant during the course of the fermentation.

The glucose concentration is kept above 1g/L. After 4 days the reactor is filled to 150 L with fresh medium. After day 9 the batch is extended by adding 1875 mL of a nutrient concentrate containing amino acids, a carbohydrate and a plant derived peptone. After 10 days another 1875 mL of the nutrient concentrate are added. Two days later (day 12) the supernatant containing erythropoietin is harvested.

**Example 9 - Production of erythropoietin in a bioreactor without methotrexate**

The CHO cell line 6C2 is cultivated in Fed-Batch (Kamp 4 B5-1 and 2) mode in a 5-L bioreactor. The medium is as in Example 9. The first bioreactor (Kamp 4 B5-1) is set up with 1.54 $\mu$m MTX in the medium, and the second (Kamp 4 B5-2) without MTX.

The glucose concentration is kept above 1g/L. The cells are seed at around $5x10^5$ cells/mL in 1250-mL medium. The $pO_2$ is set to 50% air saturation, temperature to 37°C and the pH to 7.0 and kept constant during the course of the fermentation. After 2 days the reactor is filled to 5 L with fresh medium. After day 6, 7, 8, 9 and 10 the batch is extended by adding 50 to 122-mL of a nutrient concentrate containing amino acids, a carbohydrate and a plant derived peptone. On day 11 the supernatant containing erythropoietin is harvested.

The cultivation without methotrexate is found to be superior due to the better glycosylation pattern.

**Example 10 - Production of Erythropoietin in a bioreactor with enriched medium**

The CHO cell line 6C2 is cultivated in Fed-Batch (Kamp 11 B5-1 and 2) mode in a 5 L bioreactor. Bioreactor 1 is operated as in Example 10 (Kamp 4 B5-2). In bioreactor 2 a cell culture medium is used consisting of an enriched amino acid supplemented 50:50 DMEM/Hams F12 and containing a 0.325% of a plant peptide, 0.1% lutrol, 6.4 g/L glucose, 2.5 g/L $NaHCO_3$, ethanolamine, ferric-citrate, ascorbic acid and sodium selenite and 0.6 g/L phosphate. The cells are seed at around $5x10^5$ cells/mL in 1250 mL medium. The $pO_2$ is set to 50% air saturation, temperature to 37°C. The pH is set to 7.1 at the beginning. During the course of the fermentation it is reduced step wise to 6.9.

Over the course of the cultivation the glucose concentration in bioreactor 2 is kept between 3 to 4 g/L. After 2.5 days the reactor is filled to 5 L with fresh medium. After day 6, 7, 8, 9 and 10 the batch is extended by adding an enriched nutrient concentrate containing amino acids, a carbohydrate and a plant derived peptone. On day 11 the supernatant containing Epo is harvested.

The cultivation with a nutrient enriched medium (amino acid, glucose, plant peptone and phosphate) as well as the pH-shift from 7.1 to 6.9 is found to more than double the final Epo concentration at a comparable glycosylation profile.

**Example 11 - Production of erythropoietin in a bioreactor lacking components derived from animals**

The CHO cell line 6C2 is cultivated in Fed-Batch (Kamp 17 B5-1 and 3) mode in a 5-L bioreactor. All parameters are set as in Example 10 (Kamp 4 B5-2) if not otherwise noted. In bioreactor 2, a cell culture medium is used which does not contain any components derived from animals. For example the amino acid tyrosine or cysteine, which are typically derived from an animal (like salmon or human hair) have been replaced by synthetic amino acids.

After 2.5 days the reactor is filled to 5 L with fresh medium. After day 5, 6, 7, 8 and 9 the batch is extended by adding a nutrient concentrate containing amino acids, a carbohydrate and a plant derived peptone. On day 9 to 10 the supernatant containing erythropoietin is harvested.

A medium not containing any components of animal origin is found to yield a comparable final Epo concentration. However the culture grows slower and needs an additional nutrient concentrate addition.

**Example 12 - Production of Erythropoietin in a bioreactor with enriched medium (vitamins, trace elements)**

The CHO cell line 6C2 is cultivated in Fed-Batch (Kamp 12 C) mode in a 10 L bioreactor. The bioreactor is operated as in Example 11 (Kamp 11 B5-2) with the following exceptions:

A cell culture medium is used consisting of an enriched amino acid supplemented 50:50 DMEM/Hams F12 and containing a 0.325% of a plant peptide, 0.1% lutrol, 6.4 g/L glucose, 2.5 g/L $NaHCO_3$, ethanolamine, ferric-citrate, vitamins, trace elements and sodium selenite and 0.6 g/L phosphate. The content in the concentrate is doubled and enriched with vitamins.

The cells are seeded at around $5x10^5$ cells/mL in 4500 mL medium. The $pO_2$ is set to 50% air saturation, temperature to 37°C. The pH is set to 7.1 at the beginning. During the course of the fermentation it is reduced step wise to 6.9.

Over the course of the cultivation the glucose concentration in the bioreactor is kept between 3 to 4 g/L. After 3 days the reactor is filled to 10 L with fresh medium. After day 6, 7, 8, 9, 10, 11 and 12 the batch is extended by adding the enriched nutrient concentrate. On day 13 the supernatant containing erythropoietin is harvested.

**Example 13 - Isolation of Epo**

**Cell Separation**

Recombinant human erythropoietin is produced in a Chinese hamster ovary cell line (CHO) under serum free conditions by discontinuous fed batch fermentation. After fermentation (4x ca. 1+2 batch mode expansion stages in 2 different bioreactors) the harvest broth with about 200 - 300 mg rhEpo per L is cooled down to 2-8°C and without any interim storage period clarified first by centrifugation via disc stack separator then subsequently by depth (PP Polygard 0,1µm, Seitz Bio10 or Cuno A90M08, throughput ca. 300 L/m$^2$ filter area) and 0.2µ filtration (Sartobran P, Sartorius or Duropore 0.22µ, Millipore).To avoid high cell lysis and consequently high product contamination with HCPs (host cell proteins) it is important first to harvest at an optimal time point (ca. 12 days in main culture, oxygen consumption stagnant) and second to use a cell separation equipment specially designed for separation of fragile eukaryotic cells e.g. CSC6 (6000 m$^2$ ECA, 15500 xg, ca. 200 Uh, Westfalia) with hydrohermetic feed inlet or BTPX 250 (11000 m$^2$ ECA, 13000 xg, 300 Uh, Alfa Laval) with gentle disc inlet and porcupine outlet. Comparison of different separation techniques including tangential flow filtration and centrifugation reveals differences in the cell lysis by shear stress (measured by release of the intracellular marker enzyme LDH ). The centrifugation gives the most gentle separation (<3U LDH/mg rhEpo) and is to be preferred.

In the alternative, only the centrifugation via disc stack separator (without the depth filtration step) as described above is used for the cell separation step. In another alternative, the depth filtration step (without the centrifugation step) as described above is used.

## Capture by Anion Exchange (AEX) Chromatography

After clarification the crude supernatant is diluted with approx. 3 vol. of water to reach a final conductivity of less than or equal to 5 mS/cm and adjusted to pH 7.5 with Tris base, before applying on the AEX-capture resin.

The used AEX-column has a bed height of about 10 - 20 cm and is packed with a Q Ceramic HyperD F (Biosepra) with good flow characteristics. It is equilibrated with 20 mM Tris pH 7,5 and 50 mM NaCl. The diluted cell supernatant is then loaded (10 -15 mg rhEpo per mL resin) on the column at a flow rate of 4 - 8 cm/min and the column is washed with 10 - 15 column volumes (CV) with equilibration buffer. The product is eluted by step elution achieved by changing to a higher conductivity buffer, 20 mM Tris pH 7,5 with 150 mM NaCl. The peak fractions are pooled and give a yield of about 50-60%.

In an alternative, the fraction pool is concentrated by ultrafiltration to reduce the intermediate volume and to standardise the following precipitation conditions. Preferably a 5 to 10 kDa cutoff membrane is used and a target product concentration of about 20 mg/ml is adjusted.

## Ammonium Sulfate Precipitation

The capture pool from the previous step is typically further purified by precipitation of the contaminating host cell proteins with 2.4 M (NH$_4$)$_2$SO$_4$. At this AS-concentration almost no product is found in the precipitate leaving a pure HCP free supernatant which has to be diluted before the following RPC purification to < 240 mM (NH$_4$)$_2$SO$_4$ in the RPC load.

The precipitation is performed by adding 1.5 volumes of an ammonium sulfate -stock solution (4M (NH$_4$)$_2$SO$_4$, 20mM Tris pH 7.5) to one volume of capture pool, incubation for 30 min at 10-15°C and separation of the precipitate by depth (Seitz Bio10 or Cuno A90M08) and 0,2µ filtration (Sartobran P, Sartorius or Duropore 0.22µ, Millipore) . In case of high elution volume = dilute rhEpo elution pool (< 5 mg rhEpo/ml) the HCP precipitation can be improved by an optional UF-concentration step (10 kDa cutoff).

The ammonium sulfate precipitation is more effective than hydrophobic interaction chromatography.

To reduce the ammonium sulphate content from the prior precipitation step the supernatant containing the product has to be diluted as mentioned above. An alternative is a ultrafiltration/diafiltration step. Preferably a 5 to 10 kDa cutoff membrane is used and a target ammonium sulphate concentration of less than 240 mM and a product concentration of about 30 mg/ml is adjusted. The used buffer for the diafiltration step is 20 mM Tris/HCl pH 7.0.

## Reversed Phase Chromatography

The next purification step is a reversed phase chromatography which is useful in several respects: a) the different isoforms are well separated according to their sugar backbone (highly glycosylated/sialylated elute before less glycosylated/sialylated forms), b) residual host cell proteins are removed with this high performance chromatography and c) the chromatography is a robust step for virus removal as well as for virus inactivation by the organic solvent. Source 30RPC (Amersham Biosciences) is a polymeric resin which can be a) run under medium pressure (<10 bar) and b) sanitized with high concentrations of NaOH. The preferred bed height is between 10 and 15 cm, the recommended load range 8-12 mg rhEpo per ml packed resin.

Before the RPC-step the product containing supernatant needs to be adjusted to a final concentration of less than 0.24 M ammonium sulfate. This conditioning can be performed a) by a subsequent online-dilution step (1 vol rhEpo-supernatant + 4 vol 20 mM Tris/HCl pH7.0+ 5 vol 50 w% ACN in 20 mM Tris/HCl pH 7.0 during the RPC load or to save the expansive organic solvent preferentially again by diafiltration/ concentration (UF with 10 kDa cutoff) against 20 mM Tris/HCl pH7.0 before the loading step. The column has been equilibrated before and washed after the load with 25w% acetonitrile (ACN) in 20 mM Tris pH 7.0. The product is eluted with a linear gradient from 25% to 50% ACN and collected in small fractions (in particular approx. 0.2 CV, in the alternative approx. 0.3-0.5 CV) pre-filled with 4 volumes of dilution buffer (50 mM Tris pH 7,0) to immediately reduce the solvent concentration, which may induce aggregation and impairs the following AEX chromatography. The fractions of approximately the first half of the elution peak are pooled to give the RPC-pool, which is further processed. This pool contains the isoforms with the favored higher degree of glycosylation. By this fractionation regime, des-O-glycosylated rhEpo product in which the O-glycan is missing at position Ser126, which is present in the cell culture at levels of up to 20%, is removed.

In an alternative method, to induce virus inactivation by exposure to an organic solvent, the fractions are prefilled with 0.5 volumes of 20 mM Tris pH 7.0 instead of 4 volumes of the same buffer as described above and incubated for 20 to 40 minutes or longer. After this incubation step another 3.5 volumes of 20 mM Tris pH 7.0 referring to the original undiluted fraction volume are added and thus, the virus inactivation is stopped.

The fractions with or without virus inactivation are pooled for further purification. Pooling usually starts at 50 - 100% of the maximal OD on the ascending site and ends approx. with fractions above 70-80% on the descending site. Earlier eluting fractions can contain host cell proteins, whereas later eluting fractions contain less sialylated, less active isoforms. Additionally a CZE analysis can be performed to support pooling for certain isoforms.

## Anion Exchange Chromatography

The diluted RPC-pool is then loaded on a high performance AEX column, which again helps to select for specific isoforms and remove host cell proteins. This time the isoforms are separated according to the isoelectric point, i.e. according to the number of sialic acids which is proportional to the grade of glycosylation. A high performance resin is used, Q-Sepharose HP (Amersham Biosciences), which shows excellent separation efficiency. The bed height is between 15 and 20 cm. All conditions, such as load, gradient and bed height are defined to keep a rather low product concentration, which otherwise leads to a significant post-peak during elution caused by solvent induced aggregation of the product.

The RPC-pool is loaded with 2-4 mg Epo per mL resin on a Q Sepharose HP equilibrated with 20 mM Tris pH 7,0. After a wash step with equilibration buffer, the product is eluted in a 10 CV linear salt gradient from 0 to 300 mM NaCl in equilibration buffer. The elution peak is collected in 0.1 CV or in 0.25 CV fractions and analytical pools are analyzed by CZE to find the right fraction pool, which contains the desired erythropoietin isoforms. The AEX-pool consists typically of the second half of the elution peak, where the highly glycosylated and sialylated isoforms elute.

By using the capillary zone electrophoresis (CZE) as in-process control, it is possible to produce a precisely defined mixture of erythropoietin isoforms even if the source contains only a few highly sialylated isoforms, due to different fermentation conditions or host systems.

The CZE is a high resolution method capable of separating isoforms of different charge. It gives quantitative results on every single isoform in each fraction. This information enables pooling of specific fractions leading to a consistent isoform profile from batch to batch. Typically the early eluting, less sialylated isoforms are omitted by pooling only the later eluting fractions.

## Size Exclusion Chromatography

In a last chromatographic step the AEX-pool is polished by size exclusion, which removes potential dimers and higher aggregates, and performs a buffer exchange for the final formulation. The Superdex 75 prep grade (Amersham Biosciences) used in this step has a good resolution even at higher load volumes up to 15% of the column volume. The preferred bed height is between 60 and 80 cm.

As it is not possible to run the AEX-chromatography of the previous step in a way to achieve high product concentration in the AEX-pool, the pool has to be concentrated before the gel filtration. This is performed by an ultra-filtration step using a 5 -10 kDa UF-membrane leading in an about 10 fold concentrated UF-retentate with approx. 10 mg erythropoietin per mL.

About 3-7 CV% of UF-retentate are directly loaded onto a Superdex 75 pg column preequilibrated with 20 mM Na-phosphate pH 7.0, 75 mM NaCl. After approx. 1 - 1.5 CV the product starts to elute from the column and the elution peak is collected to give the SEC-pool.

## Nano-Filtration

To remove a potential virus load an additional dead-end virus-filtration step is implemented. This filtration is performed with a special membrane, designed to remove particles as small as 15 nm, such as the Planova 15N (Asahi). Alternative dead-end nanofiltration units are PALL Ultipor VF Grade DV20 or Millipore Viresolve NFP cartridges or capsules. Especially for small non-enveloped viruses, e.g. parvovirus, there is almost no other tool of virus removal or inactivation.

The sterile filtered SEC-pool is passed over a dead-end filter with a suitable membrane and the filtrate represents the final bulk drug substance. Alternatively the nano-filtration can be inserted between the UF-concentration and the size exclusion chromatography.

## Table 1: recombinant CHO-cells: transfections with Epo/neo and Epo/dhfr

### T25 transfections

| transfection | number of clones per T25 | Qp, $9.6 \times 10^{-8}$ M MTX [$\mu g/10^6$cells/d] |
|---|---|---|
| 09/T25/1 (50:1) | 136 | 0.16 |
| 09/T25/2 (50:1) | 107 | 2.6 |
| 09/T25/3 (5:1) | 459 | 0.14 |
| 09/T25/4 (5:1) | 648 | 0.9 |

### 96-well transfections

| transfection | number of clones per plate | selected clone |
|---|---|---|
| 09/96/1 (50:1) | 47 | 1F5 |
| 09/96/2 (50:1) | 46 | |
| 09/96/3 (50:1) | 50 | 5D5, 3H5 |
| 09/96/4 (50:1) | 52 | |
| 09/96/5 (50:1) | 49 | 5D4, 5H4 |
| 09/96/6 (5:1) | 416 | 6C5 |
| 09/96/7 (5:1) | 556 | 7E9 |
| 09/96/8 (5:1) | 392 | |
| 09/96/9 (5:1) | 427 | |
| 09/96/10 (5:1) | 352 | |
| 09/96/11 (75:1) | 49 | |
| 09/96/12 (75:1) | 60 | 12A9 |

## Amplification of different clones

| clone | Qp [µg/10cells/d] | | |
|---|---|---|---|
| | $9.6 \times 10^{-8}$ M MTX | $1.9 \times 10^{-7}$ M MTX | $3.8 \times 10^{-7}$ M MTX |
| 09/96/1F5 | 11 | 11.4 | 17.1 |
| 09/96/3D5 | 8.4 | 14.4 | 11.4 |
| 09/96/3H5 | 8.2 | 14.1 | 12.9 |
| 09/96/5D4 | 4.9 | 3.8 | 3.6 |
| 09/96/5H1 | 4.7 | 7.1 | 6.7 |
| 09/96/6C5 | 4.2 | 3.8 | 3.6 |
| 09/96/7E9 | 5.5 | 8.8 | 8.9 |
| 09/96/12A9 | 6 | | |

Table 2: Subcloning conditions and plating efficiencies of cell-pool 3D5 with 0.38 µM MTX

| Number of 96 well plates | Number of seeded cells/well | % growing wells | % single clones |
|---|---|---|---|
| 25 (no. 6-30) | 10 | 13% | 77% |
| 5(no. 1-5) | 20 | 24% | 54% |

Table 3: Subcloning conditions and plating efficiencies of Subclone 3D5/1H9 and 3D5/18E5 with 1.54 µM MTX
3D5/1H9

| Number of 96 well plates | Number of seeded cells/well | % growing wells | % single clones |
|---|---|---|---|
| 8 (no. 9-16) | 10 | 6.4% | 85% |
| 8 (no. 1-8) | 30 | 19% | 46% |

3D5/18E5

| Number of 96 well plates | Number of seeded cells/well | % growing wells | % single clones |
|---|---|---|---|
| 8 (no. 9-16) | 4 | 15% | 56% |
| 8 (no. 1-8) | 8 | 29% | 44% |

Table 4: Specific productivity and growth properties of recombinant CHO-clones

| | 4C2 | 6C2 | 6D4 | 15B4 | 7A6 | 15C3 |
|---|---|---|---|---|---|---|
| Inoculated cell number [x $10^5$ cells/ml] | 2.17 | 2.67 | 2.89 | 0.71 | 2.38 | 2.16 |
| Final cell number [x $10^5$ cells/ml] | 7.27 | 9.35 | 8.8 | 2.61 | 6.41 | 6.15 |
| Specific growth rate µ [days $^{-1}$] | 0.4 | 0.42 | 0.37 | 0.43 | 0.33 | 0.35 |

## Claims

1. A method for recovering and purifying recombinant human erythropoietin (rhEpo) from a cell culture medium comprising host cells, which method comprises the steps of:

(a) removing host cells, cellular constituents and debris from the cell culture medium by performing a procedure selected from the group consisting of (i) centrifugation followed by a depth filtration step, (ii) a depth filtration step, and (iii) centrifugation, to obtain a clarified culture medium supernatant;

(b) adjusting the conductivity of the supernatant to 5 mS/cm or less, and a pH of between about 7.0 and 8.0;

(c) applying the supernatant from step (b) to a column comprising an anion exchange chromatographic medium, washing the column, eluting the rhEpo from the column, and collecting the peak fraction(s) that contain rhEpo;

(d) subjecting the combined peak fractions from step (c) to a reverse phase chromatography step using a resin that can be run under medium pressure (< 10 bar) and is resistant to high concentrations of NaOH, the rhEpo being eluted using a linear gradient of an organic solvent;

(e) applying one or more fractions eluted in step (d) which contain rhEpo to a column comprising anion exchange chromatographic media, washing the column, and eluting the rhEpo using a linear salt gradient;

(f) selecting one or more fractions eluted in step (e) which contain rhEpo based on degree of sialylation of the rhEpo; and

(g) subjecting one or more fractions eluted in step (f) which contain rhEpo by one or more size exclusion chromatographic steps using a gel filtration medium to remove potential dimers and higher aggregates; and collecting the eluate containing rhEpo.

2. The method of claim 1, wherein the centrifugation in step (a) is performed using a disc stack separator.

3. The method of claim 1 further comprising, between step (d) and step (e), the additional step of:

   selecting one or more fractions eluted in step (d) which contain rhEpo based on degree of sialylation of the rhEpo.

4. The method of claim 1 wherein prior to step (d), the peak fractions from step (c) are subjected to an ammonium sulfate precipitation step to precipitate contaminating host cell proteins, the ammonium sulfate concentration of the supernatant then being adjusted to a concentration compatible with step (d).

5. The method of claim 4 wherein said concentration is less than 0.24 M ammonium sulfate.

6. The method of claim 1 wherein the pH in step (b) is about pH 7.5.

7. The method of claim 1 wherein the elution step in step (c) is performed using a buffer having a salt concentration of greater than 125 mM.

8. The method of claim 1 wherein the organic solvent In step (d) is selected from the group consisting of acetonitrile, ethanol and hexylene glycol.

9. The method of claim 1, wherein the Epo polypeptide in step (d) is eluted employing a linear gradient of the organic solvent of from about 10% to about 100%.

10. The method of claim 8, wherein the organic solvent in step (d) is acetonitrile and the Epo polypeptide is eluted employing a gradient of from about 25% to about 50%.

11. The method of claim 1, wherein prior to step (e) the fractions eluted in step (d) are diluted with an appropriate aqueous buffer.

12. The method of claim 11, wherein prior to step (e) the diluted fractions are left for an appropriate amount of time sufficient as to inactivate viral contaminants.

13. The method of claim 1, wherein in step (g), the fractions are subjected to an ultrafiltration step prior to the size exclusion chromatography step.

14. The method of claim 1 or claim 4, wherein in step (d) the fractions are subjected to an ultrafiltration step prior to reverse phase chromatography.

15. The method of claim 4, wherein the combined peak fractions from step (c) are subjected to an ultrafiltration step prior to the ammonium sulfate precipitation step.

16. The method of claim 1 which further comprises a dead-end nano-filtration step to remove viruses before or after step (g).

17. The method of claim 1, wherein in step (a) any of the procedures (i), (ii) or (iii) is followed by a 0.2 μm filtration step.

18. The method of claim 1 or 3, wherein one or more of the selection procedure in step (f) or between step (d) and step (e) is performed by using capillary zone electrophoresis.

**Patentansprüche**

1. Verfahren zur Gewinnung und Reinigung von rekombinantem humanem Erythropoetin (rhEpo) aus einem Wirtszellen umfassenden Zellkulturmedium, wobei das Verfahren die Schritte umfasst:

   (a) Abtrennen von Wirtszellen, Zellbestandteilen und Zelltrümmern aus dem Zellkulturmedium durch einen Arbeitsschritt, der ausgewählt ist aus der Gruppe bestehend aus (i) Zentrifugation mit einem anschließenden Tiefenfiltrationsschritt, (ii) einem Tiefenfiltrationsschritt und (iii) Zentrifugation, um einen geklärten Kulturmediumüberstand zu erhalten;
   (b) Einstellen der Leitfähigkeit des Überstands auf 5 mS/cm oder weniger und eines pH-Werts zwischen etwa 7,0 und 8,0.
   (c) Aufbringen des Überstands von Schritt (b) auf eine Säule, die ein Anionenaustauschchromatographiemedium umfasst, Waschen der Säule, Eluieren des rhEpo von der Säule und Sammeln der Peakfraktion(en), die rhEpo enthalten;
   (d) Umkehrphasenchromatographie der vereinigten Peakfraktionen von Schritt (c) mit einem Harz, das unter mittlerem Druck (< 10 bar) verwendet werden kann und gegen hohe NaOH-Konzentrationen beständig ist, wobei das rhEpo mit einem linearen Gradienten eines organischen Lösemittels eluiert wird;
   (e) Aufbringen ein oder mehrerer in Schritt (d) eluierter Fraktionen, die rhEpo enthalten, auf eine Säule, die Anionenaustauschchromatographiemedien umfasst, Waschen der Säule und Eluieren des rhEpo mit einem linearen Salzgradienten;
   (f) Auswählen ein oder mehrerer in Schritt (e) eluierter Fraktionen, die rhEpo enthalten, auf Basis des Sialylierungsgrades des rhEpo; und
   (g) Unterziehen von ein oder mehreren in Schritt (f) eluierten Fraktionen, die rhEpo enthalten, ein oder mehreren Größenausschlusschromatographieschritten mit einem Gelfiltrationsmedium zur Entfernung potentieller Dimere und höherer Aggregate; und Auffangen des rhEpo enthaltenden Eluats.

2. Verfahren nach Anspruch 1, wobei die Zentrifugation in Schritt (a) mit einer Tellerzentrifuge erfolgt.

3. Verfahren nach Anspruch 1, weiterhin umfassend, zwischen Schritt (d) und Schritt (e), den zusätzlichen Schritt:

   Auswählen ein oder mehrerer in Schritt (d) eluierter Fraktionen, die rhEpo enthalten, auf Basis des Sialylierungsgrades des rhEpo.

4. Verfahren nach Anspruch 1, wobei die Peakfraktionen aus Schritt (c) vor Schritt (d) einem Fällungsschritt mit Ammoniumsulfat unterzogen werden, um kontaminierende Wirtszellproteine auszufällen, wobei die Ammoniumsulfatkonzentration des Überstands anschließend auf eine Konzentration eingestellt wird, die mit Schritt (d) kompatibel ist.

5. Verfahren nach Anspruch 4, wobei die Konzentration weniger als 0,24 M Ammoniumsulfat beträgt.

6. Verfahren nach Anspruch 1, wobei der pH in Schritt (b) etwa 7,5 beträgt.

7. Verfahren nach Anspruch 1, wobei der Elutionsschritt in Schritt (c) mit einem Puffer mit einer Salzkonzentration von mehr als 125 mM durchgeführt wird.

8. Verfahren nach Anspruch 1, wobei das organische Lösemittel in Schritt (d) ausgewählt ist aus der Gruppe bestehend aus Acetonitril, Ethanol und Hexylenglycol.

9. Verfahren nach Anspruch 1, wobei das Epo-Polypeptid in Schritt (d) mit einem linearen Gradienten des organischen Lösemittels von etwa 10% bis etwa 100% eluiert wird.

**10.** Verfahren nach Anspruch 8, wobei das organische Lösemittel in Schritt (d) Acetonitril ist und das Epo-Polypeptid mit einem Gradienten von etwa 25% bis etwa 50% eluiert wird.

**11.** Verfahren nach Anspruch 1, wobei die in Schritt (d) eluierten Fraktionen vor Schritt (e) mit einem geeigneten wässrigen Puffer verdünnt werden.

**12.** Verfahren nach Anspruch 11, wobei die verdünnten Fraktionen vor Schritt (e) für eine angemessene Dauer, die ausreicht, um virale Kontaminanten zu inaktivieren, stehen gelassen werden.

**13.** Verfahren nach Anspruch 1, wobei die Fraktionen in Schritt (g) vor dem Schritt der Größenausschlusschromatographie einem Ultrafiltrationsschritt unterzogen werden.

**14.** Verfahren nach Anspruch 1 oder Anspruch 4, wobei die Fraktionen in Schritt (d) vor der Umkehrphasenchromatographie einem Ultrafiltrationsschritt unterzogen werden.

**15.** Verfahren nach Anspruch 4, wobei die vereinigten Peakfraktionen von Schritt (c) vor dem Fällungsschritt mit Ammoniumsulfat einem Ultrafiltrationsschritt unterzogen werden.

**16.** Verfahren nach Anspruch 1, welches weiterhin vor oder nach Schritt (g) einen Dead-End-Nanofiltrationsschritt umfasst, um Viren zu entfernen.

**17.** Verfahren nach Anspruch 1, wobei einem der Arbeitsschritte (i), (ii) oder (iii) in Schritt (a) ein 0,2 $\mu$m-Filtrationsschritt folgt.

**18.** Verfahren nach Anspruch 1 oder 3, wobei ein oder mehrere der Auswahlschritte in Schritt (f) oder zwischen Schritt (d) und Schritt (e) mittels Kapillarzonenelektrophorese durchgeführt werden.

**Revendications**

**1.** Procédé de récupération et de purification d'une érythropoïétine humaine recombinante (rhEpo) à partir d'un milieu de culture cellulaire comprenant des cellules hôtes, ledit procédé comprenant les étapes suivantes qui consistent à :

(a) récupérer des cellules hôtes, des constituants et débris cellulaires depuis le milieu de culture cellulaire en réalisant une procédure choisie à partir du groupe composé (i) d'une centrifugation suivie par une étape de filtration en profondeur, (ii) d'une étape de filtration en profondeur et (iii) d'une centrifugation, afin d'obtenir un tensioactif de milieu de culture clarifié ;
(b) ajuster la conductivité du tensioactif à 5 mS/cm ou moins, et le pH à une valeur comprise entre 7,0 et 8,0 ;
(c) appliquer le tensioactif de l'étape (b) sur une colonne comprenant un milieu pour chromatographie échangeuse d'anions, laver la colonne, éluer la rhEpo depuis la colonne et collecter une (des) fraction(s) crête(s) qui contient/contiennent la rhEpo ;
(d) soumettre les fractions crêtes combinées de l'étape (c) à une étape de chromatographie à polarité de phase inversée à l'aide d'une résine qui peut être employée sous une pression moyenne (< 10 bars) et qui résiste à de fortes concentrations de NaOH, la rhEpo étant éluée à l'aide d'un gradient linéaire d'un solvant organique ;
(e) appliquer une ou plusieurs fractions éluées lors de l'étape (d) qui contiennent la rhEpo sur une colonne comprenant un milieu pour chromatographie échangeuse d'anions, laver la colonne et éluer la rhEpo à l'aide d'un gradient de salinité linéaire ;
(f) sélectionner une ou plusieurs fractions éluées lors de l'étape (e) qui contiennent la rhEpo en fonction du degré de sialylation de la rhEpo ; et
(g) soumettre une ou plusieurs fractions éluées lors de l'étape (f) qui contiennent la rhEpo à une ou plusieurs étapes de chromatographie par exclusion de tailles à l'aide d'un milieu de filtration à base d'un gel afin d'éliminer les éventuels dimères et les agrégats supérieurs ; et collecter l'éluat contenant la rhEpo.

**2.** Procédé selon la revendication 1, **caractérisé en ce que** la centrifugation de l'étape (a) est réalisée à l'aide d'un séparateur d'empilement de disques.

**3.** Procédé selon la revendication 1 comprenant, entre l'étape (d) et l'étape (e), l'étape supplémentaire qui consiste à :

sélectionner une ou plusieurs fractions éluées lors de l'étape (d) qui contiennent la rhEpo en fonction du degré de sialylation de la rhEpo.

4. Procédé selon la revendication 1, **caractérisé en ce que** préalablement à l'étape (d), les fractions crêtes de l'étape (c) sont soumises à une étape de précipitation du sulfate d'ammonium afin de précipiter les protéines des cellules hôtes contaminantes, la concentration en sulfate d'ammonium du tensioactif étant ensuite ajustée à une concentration compatible avec l'étape (d).

5. Procédé selon la revendication 4, **caractérisé en ce que** ladite concentration est inférieure à 0,24 M de sulfate d'ammonium.

6. Procédé selon la revendication 1, **caractérisé en ce que** le pH lors de l'étape (b) est d'environ 7,5.

7. Procédé selon la revendication 1, **caractérisé en ce que** l'étape d'élution lors de l'étape (c) est réalisée à l'aide d'un tampon présentant une concentration en sel supérieure à 125 mM.

8. Procédé selon la revendication 1, **caractérisé en ce que** le solvant de l'étape (d) est sélectionné à partir du groupe composé d'acétonitrile, d'éthanol et d'hexylèneglycol.

9. Procédé selon la revendication 1, **caractérisé en ce que** le polypeptide d'Epo de l'étape (d) est élué à l'aide d'un gradient linéaire du solvant organique allant d'environ 10% à environ 100%.

10. Procédé selon la revendication 8, **caractérisé en ce que** le solvant organique lors de l'étape (d) est de l'acétonitrile et **en ce que** le polypeptide d'Epo est élué à l'aide d'un gradient allant d'environ 25% à environ 50%.

11. Procédé selon la revendication 1, **caractérisé en ce que**, préalablement à l'étape (e), les fractions éluées lors de l'étape (d) sont diluées avec un tampon aqueux approprié.

12. Procédé selon la revendication 11, **caractérisé en ce que** préalablement à l'étape (e), les fractions diluées sont laissées de côté pendant un laps de temps approprié de façon à inactiver les contaminants viraux.

13. Procédé selon la revendication 1, **caractérisé en ce que** lors de l'étape (g), les fractions sont soumises à une étape d'ultra-filtration préalablement à l'étape de chromatographie par exclusion de tailles.

14. Procédé selon la revendication 1 ou 4, **caractérisé en ce que** lors de l'étape (d), les fractions sont soumises à une étape d'ultra-filtration préalablement à l'étape de chromatographie à polarité de phase inversée.

15. Procédé selon la revendication 4, **caractérisé en ce que** les fractions crêtes de l'étape (c) sont soumises à une étape d'ultra-filtration préalablement à l'étape de précipitation du sulfate d'ammonium.

16. Procédé selon la revendication 1, qui comprend une étape de nano-filtration frontale afin d'éliminer les virus avant ou après l'étape (g).

17. Procédé selon la revendication 1, **caractérisé en ce que** lors de l'étape (a), n'importe laquelle des procédures (i), (ii), ou (iii) est suivie par une étape de filtration de 0,2 um.

18. Procédé selon la revendication 1 ou 3, **caractérisé en ce qu'**une ou plusieurs des procédures de sélection lors de l'étape (f) ou entre l'étape (d) et l'étape (e) est réalisée à l'aide d'une électrophorèse capillaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9928346 A, J. Burg

### Non-patent literature cited in the description

- **TAKEUCHI ; KOBATA.** *Glycobiology,* 1991, vol. 1 (4), 337-346
- **T. MIYAKE et al.** *J. Biol. Chem,* 1977, vol. 252 (15), 5558-5564
- **N. INOUE et al.** *Biol. Pharm. Bull.,* 1994, vol. 17 (2), 180-184
- **G. KRYSTAL et al.** *Blood,* 1986, vol. 67 (1), 71-79
- **H. YIANAGI et al.** *J. Chromatogr.,* 1987, vol. 417, 178-182
- **V. BROUDY et al.** *Arch. Biochem. Biophys.,* 1988, vol. 265 (2), 329-336
- **A.B. GHANEM.** *Prep. Biochem.,* 1994, vol. 24 (2), 127-142
- **A. GOKANA et al.** *J. Chromatogr.,* 1997, vol. 791, 109-118
- **HAMMERLING et al.** *J Pharm Biomed Anal,* 1996, vol. 14 (11), 1455-69
- **SAMBROOK et al.** Molecular Cloning: A Laboratory Manual. Cold Spring Harbor Laboratory Press, 1989
- **AUSUBEL et al.** Short Protocols in Molecular Biology. John Wiley & Sons, Inc, 1999
- *American Type Culture Collection (ATCC), Rockville, Md. 20852, USA,* 29 August 2001
- **URLAUB et al.** *PNAS,* 1980, vol. 77 (7), 4216-4220